# DEMANDE DE BREVET EUROPEEN

(11) **EP 3 085 725 A1**
(43) Date de publication de la demande: **26.10.2016**
(21) Numéro de dépôt: 16173607.9
(22) Date de dépôt: 05.04.2011
(51) Int. Cl.: C08G 69/40, C08G 18/48, C08G 63/66, C07D 301/02, C12P 7/08, C08G 65/08, C08G 63/672, C12P 5/02, C12P 7/10, C08L 71/02

(54) **COPOLYMÈRE À BLOCS ISSU DE MATIÈRES RENOUVELABLES ET PROCÉDÉ DE FABRICATION D'UN TEL COPOLYMÈRE À BLOCS**

(30) Priorité: 07.04.2010 FR 1052617
(62) Demande divisionnaire de: 11718452.3
(71) Demandeur: ARKEMA FRANCE, 92700 Colombes (FR)
(72) Inventeur: MALET, Frédéric, 69008 Lyon (FR); LE, Guillaume, 14200 Herouville Saint Claire (FR); JOUANNEAU, Julien, 27500 Corneville sur Risle (FR)
(74) Mandataire: Bandpay & Greuter

(57) **Abrégé**

La présente invention concerne un copolymère à blocs issu d'au moins un monomère d'oxyde d'éthylène et/ou de propylène contenant du ¹⁴C.

La présente invention a également pour objet un procédé de préparation d'un tel copolymère à blocs.

## Description

### Domaine de l'invention

La présente invention se rapporte aux polymères thermoplastiques élastomères (abrégé TPE), et notamment les polymères techniques de haute valeur ajoutée utilisés dans des secteurs variés, tels que l'électronique, l'automobile ou le sport. La présente invention concerne plus particulièrement un polymère thermoplastique élastomère issu de matières premières renouvelables. L'invention a également pour objet un procédé de fabrication d'un tel polymère thermoplastique élastomère issu de matières renouvelables.

Dans les élastomères thermoplastiques conventionnels, on trouve essentiellement des ingrédients d'origine pétrolière ou d'origine synthétique. Leurs procédés d'obtention sont parfois considérés comme polluants sur l'environnement. En effet, les matières premières utilisées pour la synthèse de ces ingrédients sont obtenues par vapocraquage ou craquage catalytique de coupes pétrolières. L'utilisation de ces matières contribue à l'augmentation de l'effet de serre. Etant donnée la diminution des réserves pétrolières mondiales, la source de ces matières premières vient peu à peu à s'épuiser.

Les matières premières issues de la biomasse sont de source renouvelable et ont un impact réduit sur l'environnement. Elles ne nécessitent pas toutes les étapes de raffinage coûteuses en énergie des produits pétroliers. La production de CO₂ est réduite de sorte qu'elles contribuent moins au réchauffement climatique.

Il apparaît donc nécessaire de disposer de procédés de synthèse non dépendants de matière première d'origine fossile, mais utilisant plutôt des matières premières d'origine renouvelable.

Aujourd'hui, les fabricants qui utilisent des TPE adoptent de plus en plus une démarche d'écoconception et sont à la recherche de plastiques de haute valeur ajoutée d'origines végétales.

Par ailleurs, sur des marchés aussi concurrentiels que celui du sport ou de l'automobile, les fabricants doivent répondre à la demande des consommateurs pour des matériaux polymères associant performances techniques et environnementales. Or ces performances dépendent à la fois des matières premières et des procédés utilisés.

La présente invention a donc pour but de fournir des TPE, qui répondent à ces exigences, à la fois en termes de propriétés mécaniques, de résistance chimique ou au vieillissement et en termes d'éthique écologique.

### Technique antérieure

Par polymère thermoplastique élastomère (TPE), on entend un copolymère à blocs comprenant, en alternance, des blocs ou segments dits durs ou rigides (au comportement plutôt thermoplastique) et des blocs ou segments dits souples ou flexibles (au comportement plutôt élastomère).

Un bloc est dit « souple » s'il présente une faible température de transition vitreuse (Tg). Par faible température de transition vitreuse, on entend une température de transition vitreuse Tg inférieure à 15 °C, de préférence inférieure à 0°C, avantageusement inférieure à -15°C, encore plus avantageusement à -30°C, éventuellement inférieure à -50°C.

Les blocs souples sont généralement à base de polyéthers (PE) choisis parmi le poly(éthylène glycol) (PEG), le poly(1,2-propylène glycol) (PPG), le poly(1,3-propylène glycol) (PO3G), le poly(tétraméthylène glycol) (PTMG), et leurs copolymères ou mélanges. Les blocs rigides sont généralement à base de polyamide, de polyuréthane, de polyester ou d'un mélange de ces polymères.

A titre d'exemple de copolymère à blocs durs et à blocs souples, on peut citer respectivement (a) les copolymères à blocs polyester et blocs polyéther (appelés aussi COPE ou copolyétheresters), (b) les copolymères à blocs polyuréthane et blocs polyéther (appelés aussi TPU abréviation de polyuréthanes thermoplastiques) et (c) les copolymères à blocs polyamide et blocs polyéther (appelés aussi PEBA selon l'IUPAC).

Dans le domaine des élastomères thermoplastiques tels que les TPU ou les COPE, des produits fabriqués partiellement à partir de matières premières d'origine renouvelable ont été commercialisés récemment. A titre d'exemple, les TPU commercialisés sous la marque Pearlthane® ECO-D12T95 par Merquinsa revendiquent un taux de carbone renouvelable de 38% selon l'ASTM-D6866. On peut également citer la gamme de COPE commercialisée sous la marque Hytrel® RS par Dupont. Ce COPE comprend un PO3G issu de ressources renouvelables qui remplace les polyols pétrochimiques.

Quant aux PEBA, ils sont majoritairement obtenus à partir de matières premières d'origine fossile, qui seules permettent d'atteindre les compromis coûts/performances nécessaires à certaines applications.

En effet, les PEBA ou Polyether-Bloc-Amide, tels que ceux commercialisés par la Société Arkema sous le nom Pebax®, sont des élastomères thermoplastiques sans plastifiant qui appartiennent à la famille des polymères techniques. Ils peuvent être facilement traités par moulage par injection et extrusion de profilés ou films. Ils peuvent aussi être mis en oeuvre sous forme de filaments, fils et fibres pour tissus et non-tissés. Ils sont utilisés dans des applications de haute valeur ajoutée et en particulier :
- le sport de haut niveau, notamment comme éléments de semelles de chaussures de sprint, football, rugby, tennis, basket-ball, course à pied, ski alpin ou nordique, mais aussi dans les balles de golf, et dans bien d'autres articles de sport ;
- l'industrie, notamment comme bande de courroie transporteuse, comme produit imper-respirant ou comme additif antistatique ;
- le domaine médical, notamment comme cathéter, ballon d'angioplastie, courroie péristaltique ;
- l'automobile, notamment comme cuir synthétique, peaux, tableau de bord, élément d'airbag, etc...

Ils permettent de combiner dans un même polymère des propriétés mécaniques inégalées à une très bonne résistance au vieillissement thermique ou sous UV, ainsi qu'une faible densité. Ils permettent ainsi l'élaboration de pièces légères. En particulier, à dureté équivalente, ils dissipent moins d'énergie que les autres matériaux, ce qui leur confère une très bonne résistance aux sollicitations dynamiques en flexion ou traction, et ils présentent des propriétés de retour élastique exceptionnel.

Les PEBA à blocs polyamide et blocs PEG ont en outre des propriétés antistatiques permanentes bien connues et apportent aux films des propriétés imper-respirantes et d'étanchéité, comme décrit dans les brevets EP1046675 et EP0737709.

Depuis 2007, la gamme « Pebax® Rnew » commercialisée par Arkema, est la seule à proposer une gamme d'élastomères thermoplastiques techniques dont le taux de carbone d'origine renouvelable varie d'environ 20% à 95%, selon la teneur en polyamide d'origine renouvelable de l'élastomère thermoplastique. En effet le PEBA classique est un élastomère thermoplastique obtenu à partir de polyamide 12 (ou de polyamide 6) pour le bloc rigide et de polyéther pour le bloc souple, ces deux blocs étant fabriqués à partir de matières d'origine fossile.

Pour le bloc rigide, une première démarche pour évoluer vers des matières premières renouvelables a consisté à choisir un polyamide fabriqué à partir de matières premières d'origine végétale, tel que l'acide amino-11-undécanoïque. En effet, l'acide amino-11-undécanoïque est issu du traitement de l'huile de ricin (Castor oil, en anglais), extraite de la plante du même nom (le Ricin commun), à partir des graines de ricin. Le remplacement du PA 12 par le PA 11, obtenu par polycondensation de l'acide amino-11-undécanoïque, dans la fabrication de PEBA apporte, en plus d'un impact environnemental moindre, un matériau dont certaines propriétés sont supérieures à celles du matériau d'origine fossile. En particulier, les PEBA fabriqués à base de PA 11 ne présentent plus de seuil de plasticité lors de sollicitation sous contrainte, ce qui permet d'améliorer non seulement la résistance au choc à froid, mais également la résistance à la fatigue en flexion. La résistance thermique est également améliorée ainsi que la rigidification à froid.

Pour le bloc souple polyéther, en revanche, il n'existe pas actuellement d'alternative renouvelable autre que des blocs polyethers PO3G mis sur le marché récemment par Dupont sous la marque Cerenol®, ou alors des polyesters souples, à base d'acide gras dimérisé, commercialisés par Uniqema sous la marque Priplast®. Toutefois, l'utilisation de blocs polyéthers uniquement PO3G ou de blocs polyesters uniquement Priplast® pour la fabrication des PEBA ne permet pas d'atteindre les mêmes performances qu'avec des blocs PEG, en termes de densité, de reprise en eau et/ou de propriétés mécaniques, de propriétés antistatiques, de propriétés imper-respirantes et de propriétés d'étanchéité. Par ailleurs l'utilisation de ces seuls blocs PO3G ou polyesters ne permet pas de moduler facilement à la fois les propriétés mécaniques et antistatiques du copolymère à blocs. De plus, l'utilisation de ces blocs PO3G ne permet pas d'atteindre les mêmes performances qu'avec des blocs PPG, en termes de faible reprise en humidité, et notamment en terme de stabilité des propriétés mécaniques vis-à-vis de l'humidité ambiante.

La présente invention a donc pour but de concevoir un nouveau TPE à la fois d'origine renouvelable et de haute performance. La présente invention a notamment pour but de fournir un TPE d'origine renouvelable, mais de performances au moins équivalentes à celles des TPE classiques d'origine fossile.

La présente invention a également pour but de fournir un procédé de fabrication d'un tel TPE qui soit simple, facile à mettre en oeuvre, rapide (qui présente le moins d'étapes possibles), et qui ne nécessite pas l'intervention de manipulations chimiques ou technologiques lourdes, coûteuses en énergie ou polluantes, pour avoir un impact environnemental le plus faible possible. En particulier, la présente invention a pour but de fournir un tel procédé facilement modulable, c'est-à-dire adaptable facilement en fonction des propriétés antistatiques, antiperspirantes, et mécaniques souhaitées.

Fort de son expertise dans la fabrication de polymères bioressourcés haute performance, la demanderesse a maintenant démontré qu'il était possible de fabriquer un TPE :
- dont les blocs polyether souples sont d'origine renouvelable, grâce à l'utilisation d'oxyde d'éthylène et/ou d'oxyde de propylène contenant du ¹⁴C, notamment grâce à l'utilisation de blocs PEG et/ou PPG fabriqués à partir de biomasse, et
- dont les performances sont au moins identiques à celles des TPE correspondants dont les blocs polyether sont d'origine fossile.

### Résumé de l'invention

L'invention a pour objet un copolymère à blocs issu d'au moins un monomère d'oxyde d'éthylène et/ou d'oxyde de propylène contenant du ¹⁴C . Avantageusement, ledit copolymère comprend au moins un bloc polyéther issu au moins partiellement d'oxyde d'éthylène et/ou d'oxyde de propylène contenant du ¹⁴C . Avantageusement, le copolymère à blocs selon l'invention comprend:
- de 1 à 99% d'au moins un bloc polyéther souple issu au moins partiellement d'oxyde d'éthylène et/ou d'oxyde de propylène contenant du ¹⁴C , et
- de 1 à 99% d'au moins un bloc rigide choisi parmi : les blocs polyamide, les blocs polyuréthane, les blocs polyester, et leurs mélanges.

Avantageusement, ledit au moins un bloc polyéther souple comprend au moins un polyéthylène glycol (PEG) et/ou polypropylène glycol (PPG) issu au moins partiellement de matières renouvelables. Avantageusement, la proportion massique dudit au moins un bloc souple représente de 5 à 95%, de préférence de 5 à 85%, et la proportion massique dudit au moins un bloc rigide représente de 5 à 95%, de préférence de 15 à 95%, sur la masse totale du copolymère.

Avantageusement, ledit au moins un bloc rigide est issu au moins partiellement de matières premières renouvelables.

Avantageusement, ledit au moins un bloc polyéther et/ou ledit au moins un bloc rigide est/sont issu(s) en totalité de matières renouvelables.

Avantageusement, le copolymère selon l'invention comprend une teneur en bio-carbone d'au moins 1%, qui correspond à un ratio isotopique de ¹⁴C/¹²C d'au moins 1,2. 10⁻¹⁴. Avantageusement, le copolymère selon l'invention comprend une teneur en bio-carbone supérieure à 5%, de préférence supérieure à 10%, de préférence supérieure à 25%, de préférence supérieure à 50%, de préférence supérieure à 75%, de préférence supérieure à 90%, de préférence supérieure à 95%, de préférence supérieure à 98%, de préférence supérieure à 99%, avantageusement sensiblement égale à 100% ce qui correspond à un ratio isotopique de ¹⁴C/¹²C de 1,2. 10⁻¹².

Avantageusement, le copolymère selon l'invention comprend au moins un bloc polyamide. Avantageusement, ledit polyamide comprend un copolyamide. Avantageusement, le copolymère selon l'invention comprend au moins un bloc polyamide comprenant au moins une des molécules suivantes : acide amino-11-undécanoïque, acide n-heptylamino-11-undécanoïque, acide succinique, acide azélaïque, acide sébacique, acide dodécanedioïque, acide myristique, acide tétradécanedioïque, acide hexadécanedioïque, acide octadécanedioïque, butanediamine, pentanediamine, décaméthylènediamine, diacide(s) gras, dimère(s) d'acide gras et leurs mélanges. Avantageusement, ledit au moins un bloc polyamide comprend au moins un monomère choisi parmi les monomères de polyamide suivants : 11, 5.4, 5.9, 5.10, 5.12, 5.13, 5.14, 5.16, 5.18, 5.36, 6.4, 6.9, 6.10, 6.12, 6.13, 6.14, 6.16, 6.18, 6.36, 10.4, 10.9, 10.10, 10.12, 10.13, 10.14, 10.16, 10.18, 10.36, 10.T, 12.4, 12.9, 12.10, 12.12, 12.13, 12.14, 12.16, 12.18, 12.36, 12.T et leurs mélanges ou copolymères. Avantageusement, le copolymère à blocs selon l'invention est un copolymère à blocs polyéther et blocs polyamide (abrégé PEBA).

Avantageusement, ledit PEBA est à base de PA11-PEG, PA10.10-PEG, PA10.12-PEG, PA10.14-PEG, PA6.10-PEG, PA6.12-PEG, PA6.18-PEG, PA11-PPG, PA10.10-PPG, PA10.12-PPG, PA10.14-PPG, PA6.10-PPG, PA6.12-PPG, PA6.18-PPG, PA11-PEG/PPG, PA10.10-PEG/PPG, PA10.12-PEG/PPG, PA10.14-PEG/PPG, PA6.10-PEG/PPG, PA6.12-PEG/PPG, et/ou PA6.18-PEG/PPG, de préférence à base de PA11-PEG, PA11-PPG ou PA11-PEG/PPG.

Avantageusement, le copolymère selon l'invention comprend au moins un bloc polyester. Avantageusement, ledit polyester comprend un copolymère. Avantageusement, ledit au moins un bloc polyester comprend au moins une des molécules suivantes : éthylène glycol, 1,3-propanediol, 1,4-butanediol, 1,10-décanediol, acide gras dimérisé réduit pour obtenir le diol correspondant, acide 2,5-furanedicarboxylique, acide succinique, acide azélaïque, acide sébacique, acide dodécanedioïque, acide myristique, acide tétradécanedioïque, acide hexadécanedioïque, acide octadécanedioique, et/ou acides gras dimérisés. Avantageusement, ledit copolymère selon l'invention est un polyétherester.

Avantageusement, le copolymère selon l'invention comprend au moins un bloc polyuréthane. Avantageusement, ledit polyuréthane comprend un copolymère, c'est-à-dire un copolyuréthane. Avantageusement, ledit au moins un bloc polyuréthane est fabriqué à partir d'au moins un polyol d'origine renouvelable, choisi parmi les polyols suivants : polyols issus de l'amidon ; érythritol ; sorbitol ; maltitol ; mannitol ; polyols dérivant de sucres, du saccharose ; isomalt ; le xylitol ; polyols issus du maïs, du soja, du coton, du colza, du tournesol ou de l'arachide ; glycérol ; propylène glycol ; éthylène glycol ; les coproduits de réaction de production de biodiésel ; polyéthylène glycol (PEG), poly(1,2-propylène glycol) (PPG), poly(1,3-propylène glycol) (PO3G), polytétraméthylène glycol (PTMG). Avantageusement, ledit copolymère selon l'invention est un polyétheruréthane.

Avantageusement, ledit au moins un bloc polyéther comprend en outre des polyéthers autres que PEG et/ou PPG, tels que le le PO3G, le PTMG, le poly(3-méthyltétrahydrofurane d'origine renouvelable ou d'origine fossile et/ou du PEG ou PPG d'origine fossile.

Avantageusement, le copolymère selon l'invention est un copolymère segmenté à blocs comprenant trois types de blocs différents (ci-après nommé tribloc), ledit tribloc étant choisi parmi les copolyétheresteramides, les copolyétheramideuréthanes, les copolyétheresteruréthanes, dans le(s)quel(s) :
- le pourcentage massique en bloc souple polyéther est supérieur à 20% ;
- le pourcentage massique en bloc rigide polyamide est supérieure à 10% ;
sur la masse totale de tribloc.

La présente invention a également pour objet un procédé de préparation de copolymère à blocs conforme à celui défini précédemment, comprenant l'étape de fourniture de polyéther issu au moins partiellement d'oxyde d'éthylène et/ou d'oxyde de propylène contenant du ¹⁴C et la transformation par synthèse en copolymère à blocs. Avantageusement, ledit polyéther comprend du polytéthylène glycol et/ou du polypropylène glycol ayant une teneur en biocarbone d'au moins 1%.

Avantageusement, l'étape de fourniture comprend une étape de production de polyéther à partir d'oxyde d'éthylène et/ou d'oxyde de propylène ayant une teneur en biocarbone d'au moins 1%. Avantageusement, l'étape de fourniture comprend une étape de production dudit oxyde d'éthylène et/ou oxyde de propylène respectivement à partir de d'éthylène et/ou propylène. Avantageusement, l'étape de fourniture comprend une étape de production dudit éthylène et/ou propylène à partir de biomasse végétale. Avantageusement, ledit éthylène et/ou propylène est produit par déshydratation de bioéthanol et/ou d'isopropanol.

La présente invention a également pour objet l'utilisation de copolymère à blocs conforme à celui défini précédemment, dans l'automobile, les textiles, les tissés, les non-tissés, l'habillement, les chaussures, les articles de sport, les articles de loisirs, l'électronique, le matériel informatique, les équipements de santé, les additifs industriels, l'emballage et/ou les produits ménagers.

Avantageusement, le copolymère à blocs selon l'invention est utilisé dans les tableaux de bord, les airbags, les semelles de chaussure de sport, les balles de golf, les tubes à usage médical, les cathéters, les ballons d'angioplastie, les courroies péristaltiques, les bandes de courroie transporteuse, les produits imper-respirant, les additifs antistatiques, les peaux, les cuirs synthétiques, les films thermoplastiques et/ou les films d'emballage.

Avantageusement, le copolymère à blocs de l'invention est utilisé seul ou en mélange, ledit copolymère représentant en masse de 5 à 100%, de préférence de 5 à 70%, de préférence de 5 à 30%.

### Description détaillée de l'invention :

L'invention utilise les produits d'origine naturelle comme produits de départ pour la fabrication de thermoplastiques élastomères.

Le carbone d'un biomatériau provient de la photosynthèse des plantes et donc du CO₂ atmosphérique. La dégradation (par dégradation, on entendra aussi la combustion/incinération en fin de vie) de ces matériaux en CO₂ ne contribue donc pas au réchauffement puisqu'il n'y a pas d'augmentation du carbone émis dans l'atmosphère. Le bilan CO₂ des biomatériaux est donc nettement meilleur et contribue à réduire l'empreinte carbone des produits obtenus (seule l'énergie pour la fabrication est à prendre en compte). Au contraire, un matériau d'origine fossile se dégradant aussi en CO₂ va contribuer à l'augmentation du taux de CO₂ et donc au réchauffement climatique. Les composés selon l'invention auront donc une empreinte carbone qui sera meilleure que celle de composés obtenus à partir d'une source fossile. L'invention améliore donc aussi le bilan écologique lors de la fabrication des TPE.

Le terme de "bio-carbone" indique que le carbone est d'origine renouvelable, ou d'origine naturelle et provient d'un biomatériau, comme indiqué ci-après. La teneur en biocarbone, la teneur en ¹⁴C et la teneur en biomatériau sont des expressions désignant la même valeur.

Un matériau d'origine renouvelable ou biomatériau, est un matériau organique dans lequel le carbone provient de CO₂ fixé récemment (à l'échelle humaine) par photosynthèse à partir de l'atmosphère. Sur terre, ce CO₂ est capté ou fixé par les plantes. En mer, le CO₂ est capté ou fixé par des bactéries ou du plancton procédant à une photosynthèse. Un biomatériau (100% de carbone origine naturelle) présente un ratio isotopique ¹⁴C/¹²C supérieur à 10⁻¹², typiquement de l'ordre de 1,2 x 10⁻¹², tandis qu'un matériau fossile a un ratio nul. En effet, l'isotope ¹⁴C se forme dans l'atmosphère et est ensuite intégré par photosynthèse, selon une échelle de temps de quelques dizaines d'années au plus. La demi-vie du ¹⁴C est de 5730 ans. Donc les matériaux issus de la photosynthèse, à savoir les végétaux de manière générale, ont nécessairement une teneur maximale en isotope ¹⁴C.

La présence de ¹⁴C, la teneur en biomatériau, la teneur en biocarbone, ou la teneur en carbone organique d'origine renouvelable d'un matériau est déterminée en application des normes ASTM D 6866 (ASTM D 6866-06) et ASTM D 7026 (ASTM D 7026-04). La norme ASTM D 6866 a pour objet "Determining the Biobased Content of Natural Range Materials Using Radiocarbon and Isotope Ratio Mass Spectrometry Analysis", tandis que la norme ASTM D 7026 a pour objet "Sampling and Reporting of Results for Détermination of Biobased Content of Materials via Carbon Isotope Analysis". La seconde norme renvoie dans son premier paragraphe à la première. La première norme décrit un test de mesure du ratio ¹⁴C/¹²C d'un échantillon et le compare avec le ratio ¹⁴C/¹²C d'un échantillon référence d'origine 100% renouvelable, pour donner un pourcentage relatif de C d'origine renouvelable dans l'échantillon. La norme est basée sur les mêmes concepts que la datation au ¹⁴C, mais sans faire application des équations de datation. Le ratio ainsi calculé est désigné comme le "pMC" (percent Modern Carbon). Si le matériau à analyser est un mélange de biomatériau et de matériau fossile (sans isotope radioactif), alors la valeur de pMC obtenu est directement corrélée à la quantité de biomatériau présent dans l'échantillon. La valeur de référence utilisée pour la datation au ¹⁴C est une valeur datant des années 1950. Cette année a été choisie en raison de l'existence d'essais nucléaires dans l'atmosphère qui ont introduit des grandes quantités d'isotopes dans l'atmosphère après cette date. La référence 1950 correspond à une valeur pMC de 100. Compte tenu des essais thermonucléaires, la valeur actuelle à retenir est d'environ 107,5 (ce qui correspond à un facteur de correction de 0,93). La signature en carbone radioactif d'un végétal actuel est donc de 107,5. Une signature de 54 pMC et de 99 pMC correspondent donc à une quantité de biomatériau dans l'échantillon de 50% et de 93%, respectivement.

La norme ASTM D 6866 propose trois techniques de mesure de la teneur en isotope ¹⁴C:
- LSC (Liquid Scintillation Counting) spectrométrie à scintillation liquide. Cette technique consiste à compter des particules "Bêta" issues de la désintégration du ¹⁴C. On mesure le rayonnement Bêta issu d'un échantillon de masse connue (nombre d'atomes C connu) pendant un certain temps. Cette "radioactivité" est proportionnelle au nombre d'atomes de ¹⁴C , que l'on peut ainsi déterminer. Le ¹⁴C présent dans l'échantillon émet des rayonnements ß, qui au contact du liquide scintillant (scintillateur) donnent naissance à des photons. Ces photons ont des énergies différentes (comprises entre 0 et 156 keV) et forment ce que l'on appelle un spectre de ¹⁴C. Selon deux variantes de cette méthode, l'analyse porte soit sur le CO₂ préalablement produit par l'échantillon carboné dans une solution absorbante appropriée, soit sur le benzène après conversion préalable de l'échantillon carboné en benzène. La norme ASTM D 6866 donne donc deux méthodes A et C, basées sur cette méthode LSC.
- AMS/IRMS (Accelerated Mass Spectrometry couplé avec Isotope Radio Mass Spectrometry). Cette technique se base sur la spectrométrie de masse. L'échantillon est réduit en graphite ou en CO₂ gazeux, analysé dans un spectromètre de masse. Cette technique utilise un accélérateur et un spectromètre de masse pour séparer les ions ¹⁴C des ¹²C et donc déterminer le rapport des deux isotopes.

Les composés selon l'invention proviennent au moins en partie de biomatériau et présentent donc une teneur en biomatériau d'au moins 1%, ce qui correspond à une teneur en ¹⁴C d'au moins 1,2 x 10⁻¹⁴. Cette teneur est avantageusement plus élevée, notamment jusqu'à 100%, qui correspond à une teneur en ¹⁴C de 1,2 x 10⁻¹². Les composés selon l'invention peuvent donc comprendre 100% de bio-carbone ou au contraire résulter d'un mélange avec une origine fossile.

Les composés selon l'invention sont, comme indiqué supra, des thermoplastiques élastomères (TPE) issus au moins partiellement de matières premières d'origine renouvelable.

Plus précisément, la présente invention a pour objet des élastomères thermoplastiques (TPE), copolymères à blocs, comprenant au moins un bloc polyéther issu d'au moins un monomère d'oxyde d'éthylène et/ou d'oxyde de propylène contenant du ¹⁴C.

Comme polyéthers pouvant être fabriqués à partir d'oxyde d'éthylène contenant du ¹⁴C, on peut citer le poly(oxyde d'éthylène), appelé aussi polyoxyéthylène glycol ou plus couramment appelé poly(éthylène glycol) ou polyéthylène glycol (abrégé PEG). Comme polyéthers pouvant être fabriqués à partir d'oxyde de propylène contenant du ¹⁴C, on peut citer le poly(oxyde de propylène), appelé aussi polyoxypropylène glycol ou plus couramment appelé poly(1,2-propylène glycol) ou polypropylène glycol (abrégé PPG).

Les blocs polyéther des TPE selon l'invention sont obtenus au moins partiellement à partir de matières premières d'origine renouvelable et comprennent au moins un motif -O-(CH₂)₂- et/ou au moins un motif -O-CHCH₃-(CH₂) -, dans lesquels les carbones sont des bio-carbones.

Par blocs polyéther (abrégé ci-après PE) au sens de l'invention on entend les polyalkylènes éthers polyols, notamment des polyalkylènes éther diols. Ledit au moins un bloc PE du copolymère de l'invention comprend au moins du poly(éthylène glycol) (PEG) et/ou du polypropylène glycol (PPG) d'origine au moins partiellement renouvelable, mais le ou les blocs PE du copolymère de l'invention peuvent comprendre en outre d'autres PE d'origine renouvelable ou d'origine fossile, choisis parmi le poly(éthylène glycol) (PEG) et/ou polypropylène glycol (PPG) d'origine non renouvelable, le poly(1,2-propylène glycol) (PPG), le polytétraméthylène éther glycol (PTMG), le polyhexaméthylène glycol, le poly(1,3-propylène glycol) (PO3G), les poly(3-alkyl tétrahydrofurane) en particulier le poly(3-méthyltétrahydrofurane (poly(3MeTHF)), et leurs mélanges. On peut également envisager un bloc PE de type «copolyéther» à blocs ou statistique contenant un enchaînement d'au moins deux types de PE cités ci-dessus.

Les blocs polyéther peuvent également comprendre des blocs obtenus par oxyéthylation de bisphenols, tels que par exemple le bisphenol A. Ces derniers produits sont décrits dans le brevet EP 613 919. Les blocs polyéthers peuvent aussi comprendre des aminés primaires éthoxylées. On utilise avantageusement aussi ces blocs. A titre d'exemple d'amines primaires éthoxylées on peut citer les produits de formule : 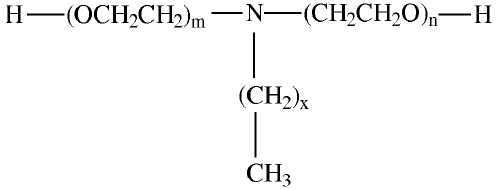 dans laquelle m et n sont compris entre 1 et 20 et x entre 8 et 18. Ces produits sont disponibles dans le commerce sous la marque NORAMOX® de la société CECA et sous la marque GENAMIN® de la société CLARIANT.

Ainsi, les fins de chaînes des blocs PE peuvent être diOH, diNH2, diisocyanate ou diacide suivant leur procédé de synthèse. Les blocs PE à bouts de chaîne NH2, peuvent être obtenus par cyanoacétylation de séquences polyoxyalkylène alpha-oméga dihydroxylées aliphatiques appelées polétherdiols telles que les Jeffamines® D300, D400, D2000, ED-600, ED-900, ED2003, les Elastamines® RP-409, RP-2009, RT-1000, RE-600, RE-900, RE-2000, HT-1700, HE-180 de la société Huntsman. De tels blocs sont décrits dans les brevets JP 2004346274, JP 2004352794 et EP1482011.

Selon un mode de réalisation, ledit au moins un polyéther, par exemple le PEG, issu d'au moins un mononomère d'oxyde d'éthylène contenant du ¹⁴C , qui est synthétisé à partir d'éthylène qui est lui-même synthétisé à partir d'éthanol (bioéthanol) ou d'un mélange d'alcools comprenant de l'éthanol. Ces alcools sont issus de la fermentation de matières premières renouvelables, notamment végétales choisies parmi la canne à sucre et la betterave sucrière, l'érable, le palmier-dattier, le palmier à sucre, le sorgho, l'agave américain, le maïs, le blé, l'orge, le sorgho, le froment, le riz, la pomme de terre, le manioc, la patate douce, les algues.

Selon un deuxième mode de réalisation, ledit au moins un polyéther, par exemple le PPG, est issu d'au moins un monomère d'oxyde de propylène (PPG) contenant du ¹⁴C , qui est synthétisé à partir de propylène qui est lui-même synthétisé à partir d'un alcool ou d'un mélange d'alcools, ledit alcool ou mélange d'alcools comprenant au moins de l'isopropanol et/ou au moins un mélange d'éthanol et de 1-butanol. Ces alcools sont eux-mêmes des dérivés de matières premières renouvelables et sont issus de la fermentation de matières végétales choisies parmi la canne à sucre et la betterave sucrière, l'érable, le palmier-dattier, le palmier à sucre, le sorgho, l'agave américain, le maïs, le blé, l'orge, le sorgho, le froment, le riz, la pomme de terre, le manioc, la patate douce, les matériaux comprenant de la cellulose ou de l'hémicellulose, tels que le bois, la paille ou le papier.

Ces deux modes de réalisation de l'invention sont alternatifs ou peuvent être combinés, de sorte que le bloc polyéther comprend à la fois du PEG et du PPG contenant du ¹⁴C, selon la norme ASTM D6866.

Les élastomères thermoplastiques (TPE) selon l'invention peuvent contenir en outre d'autres blocs souples qui ne sont pas sur base polyéther. On peut citer notamment les blocs sur base polyesters comme poly(caprolactone) ou les polyesters type Priplast®, sur base polydiméthylsiloxane ou PDMS, sur base polycarbonate aliphatique, sur base polyoléfine comme le polybutadiene, polyisobutylène etc...

En outre, les élastomères thermoplastiques (TPE) selon l'invention comprennent au moins un bloc rigide choisi parmi les blocs polyamide, les blocs polyuréthane, les blocs polyester, et leurs mélanges sous forme de copolymères à blocs ou statistiques.

Les blocs rigides peuvent être issus de matières renouvelables et/ou de matières d'origine fossile. De manière avantageuse, lesdits blocs rigides sont également issus au moins partiellement de matières renouvelables. Selon un mode particulièrement avantageux de la présente invention, les blocs polyéther et/ou les blocs polyamide et/ou les blocs polyuréthane et/ou les blocs polyester sont issus en totalité de matières renouvelables.

Ainsi, selon le choix des composants des blocs souples et rigides, la teneur en bio-carbone du copolymère de l'invention est d'au moins 1%, ce qui correspond à un ratio isotopique de ¹⁴C/¹²C d'au moins 1,2. 10⁻¹⁴. La teneur en bio-carbone du copolymère de l'invention est de préférence supérieure à 5%, de préférence supérieure à 10%, de préférence supérieure à 25%, de préférence supérieure à 50%, de préférence supérieure à 75%, de préférence supérieure à 90%, de préférence supérieure à 95%, de préférence supérieure à 98%, de préférence supérieure à 99%. Elle est avantageusement sensiblement égale à 100%, ce qui correspond à un ratio isotopique de ¹⁴C/¹²C d'au moins 1,2. 10⁻¹².

Selon un mode de réalisation préféré, le copolymère à blocs de la présente invention comprend au moins un bloc souple polyéther issu au moins partiellement d'oxyde d'éthylène et/ou d'oxyde de propylène contenant du ¹⁴C , de préférence au moins un PEG et/ou PPG obtenu(s) au moins en partie à partir de matières premières d'origine renouvelable, et des blocs polyamide.

Par polyamide au sens de l'invention on entend les homopolyamides et copolyamides ; c'est-à-dire les produits de condensation des lactames, des aminoacides ou des diacides avec les diamines et, en règle générale, tout polymère formé par des motifs reliés entre eux par des groupes amides.

Par polyamide d'origine totalement renouvelable pouvant entrer dans le copolymère selon l'invention, on entend :
- les polyamides aliphatiques obtenus à partir de lactames ou d'aminoacides (comme par exemple le PA 11 obtenu par polycondensation de l'acide amino-11-undécanoïque) ;
- les produits de condensation d'un acide dicarboxylique avec une diamine (comme par exemple le PA 10.10, produit de la condensation de la décanediamine avec l'acide sébacique, ou encore le PA 10.12 produit de la condensation de la décanediamine avec l'acide dodécanedioïque, ou encore le PA 10.36, produit de la condensation de la décanediamine avec un dimère d'acide gras) ;
- les copolyamides résultant de la polymérisation de divers monomères, tels que ceux cités ci-dessus, comme par exemple les copolyamides suivants : PA 11/10.10, PA 11/ 10.12, PA 10.10/10.12, PA 11/10.36, PA 10.12/10.36, PA 10.10/10.36, le copolyamide amino-11-undécanoïque/n-heptyl-11-aminoundécanoïque, etc. Les copolyamides d'origine renouvelable, qui comprennent au moins deux monomères, sont plus particulièrement décrits dans la demande de brevet française n° : 07.53319.

Le terme « monomère » dans la présente description des copolyamides doit être pris au sens d' « unité répétitive ». En effet, le cas où une unité répétitive du PA est constituée de l'association d'un diacide avec une diamine est particulier. On considère que c'est l'association d'une diamine et d'un diacide, c'est-à-dire le couple diamine.diacide (en quantité équimolaire), qui correspond au monomère. Ceci s'explique par le fait qu'individuellement, le diacide ou la diamine n'est qu'une unité structurale, qui ne suffit pas à elle seule à polymériser.

A titre d'exemples d'aminoacides d'origine renouvelable, on peut citer: l'acide 11-aminoundecanoïque produit à partir d'huile de ricin par exemple, l'acide 10-aminodécanoïque produit à partir de l'acide decylénique obtenu par métathèse de l'acide oléïque par exemple, l'acide 9-aminononanoïque produit à partir de l'acide oléïque par exemple.

A titre d'exemples de diacides d'origine renouvelable, on peut citer, en fonction du nombre x de carbones de la molécule (Cx) :
- C4 : l'acide succinique à partir du glucose par exemple ;
- C6 : l'acide adipique à partir du glucose par exemple ;
- C7 : l'acide heptanedioïque à partir d'huile de ricin ;
- C9 : l'acide azélaïque à partir de l'acide oléïque (ozonolyse) par exemple ;
- C10 : l'acide sébacique à partir de l'huile de ricin par exemple ;
- C11 : l'acide undécanedioïque à partir d'huile de ricin ;
- C12 : l'acide dodécanedioïque à partir de biofermentation de l'acide dodecanoïque = acide laurique (huile riche : huile de palmiste et noix de coco) par exemple ;
- C13 : acide brassylique à partir de l'acide erucique (ozonolyse) que l'on trouve dans le colza par exemple ;
- C14 : acide tetradécanedioïque par biofermentation de l'acide myristique (huile riche : huile de palmiste et noix de coco) par exemple ;
- C16 : acide hexadécanedioïque par biofermentation de l'acide palmitique (huile de palme principalement) par exemple ;
- C18 : acide octadécanedioïque obtenu par biofermentation de l'acide stéarique (un peu dans toutes les huiles végétales mais majoritaire dans les graisses animales) par exemple ;
- C20 : acide eicosanedioïque obtenu par biofermentation de l'acide arachidique (majoritaire dans l'huile de colza) par exemple ;
- C22 : acide docosanedioïque obtenu par métathèse de l'acide undécylénique (huile de ricin) par exemple
- C36 : dimère d'acide gras issu principalement des acides oléiques et linoléiques.

A titre d'exemples de diamines d'origine renouvelable, on peut citer, en fonction du nombre x de carbones de la molécule (Cx) :
- C4 : butanediamine obtenu par amination de l'acide succinique, par biofermentation ;
- C5 : pentaméthylene diamine (à partir de lysine) ;
   et ainsi de suite pour les diamines obtenues par amination des diacides d'origine renouvelable vus précédemment.

Par polyamide d'origine partiellement renouvelable, c'est-à-dire issu en partie seulement de matières renouvelables (nommé dans le texte polyamide « mixte »), on entend :
- les produits de condensation d'un acide dicarboxylique avec une diamine, et dans lesquels l'un des deux seulement (le diacide ou la diamine sont d'origine renouvelable. C'est le cas du PA 6.10 par exemple, puisque dans le monomère 6.10, seul l'acide sébacique est d'origine renouvelable tandis que l'héxaméthylène diamine est issue de la pétrochimie.
- les copolyamides résultant de la polymérisation de divers monomères (renouvelables, non renouvelables ou mixtes) tels que ceux cités ci-dessus. C'est le cas par exemple du CoPA 6.6/10.10 dans lequel le monomère « 6.6 » est d'origine non renouvelable tandis que le monomère « 10.10 » est d'origine renouvelable. C'est le cas également du PA 11/10.T par exemple, qui comporte un monomère d'origine renouvelable (« 11 ») et un monomère mixte d'origine partiellement renouvelable (« 10.T ») puisque seule la décanediamine est d'origine renouvelable, alors que l'acide téréphtalique (T) ne l'est pas.

Par polyamide d'origine fossile, on entend tous les autres polyamides de la Terre n'entrant pas dans les deux catégories précitées, c'est-à-dire qui ne sont pas fabriqués à partir de matières premières organiques renouvelables.

Avantageusement, le copolymère à blocs de la présente invention forme un polyéther bloc amide, abrégé PEBA.

Les PEBA selon l'invention incluent donc tout TPE comprenant au moins un bloc polyéther, ce dernier étant issu au moins partiellement d'oxyde d'éthylène et/ou d'oxyde de propylène comprenant du ¹⁴C, tels que le PEG et/ou le PPG respectivement issu(s) au moins partiellement de matières renouvelables, et au moins un bloc PA (homopolyamide ou copolyamide) issu de matières fossiles ou bien issu totalement ou partiellement (cas de polyamides mixtes) de matières premières renouvelables.

Les PEBA résultent de la polycondensation de blocs polyamides à extrémités réactives avec des blocs polyéthers à extrémités réactives, telles que, entre autres :
1) blocs polyamides à bouts de chaîne diamines avec des blocs polyoxyalkylènes à bouts de chaînes dicarboxyliques.
2) blocs polyamides à bouts de chaînes dicarboxyliques avec des blocs polyoxyalkylènes à bouts de chaînes diamines, obtenues par cyanoéthylation et hydrogénation de blocs polyoxyalkylène alpha-oméga dihydroxylées aliphatiques appelées polyétherdiols
3) blocs polyamides à bouts de chaînes dicarboxyliques avec des polyétherdiols, les produits obtenus étant, dans ce cas particulier, des polyétheresteramides.

Les blocs polyamides à bouts de chaînes dicarboxyliques proviennent, par exemple, de la condensation de précurseurs de polyamides en présence d'un diacide carboxylique limiteur de chaîne. Les blocs polyamides à bouts de chaînes diamines proviennent par exemple de la condensation de précurseurs de polyamides en présence d'une diamine limiteur de chaîne. La masse molaire en nombre Mn des blocs polyamides est comprise dans la gamme allant de 400 à 20000 g/mol, de préférence de 500 à 10000 g/mol, et de préférence encore de 600 et 6000 g/mol.

Les polymères à blocs polyamides et blocs polyéthers peuvent aussi comprendre des motifs répartis de façon aléatoire.

Les blocs polyamides peuvent comporter des homopolyamides ou des copolyamides. Trois types de polyamides peuvent entrer dans la composition de ces blocs PA.

Selon un premier type, les blocs polyamide proviennent de la condensation d'au moins un diacide carboxylique (aliphatique, cycloaliphatique ou aromatique) en particulier ceux ayant de 4 à 36 atomes de carbone, de préférence ceux ayant de 6 à 18 atomes de carbone et d'au moins une diamine (aliphatique, cycloaliphatique ou aromatique) choisie en particulier parmi celles ayant de 2 à 20 atomes de carbone, de préférence celles ayant de 6 à 15 atomes de carbone. A titre d'exemples de diacides aliphatiques, on peut citer les acides butanedioïque, adipique, subérique, azélaïque, sébacique, dodécanedicarboxylique, myristique, tétradécanedicarboxylique, hexadécanedicarboxylique, octadécanedicarboxylique et les acides gras dimérisés. A titre d'exemples de diacides cycloaliphatiques, on peut citer l'acide 1,4-cyclohexyldicarboxylique. A titre d'exemples de diacides aromatiques, on peut citer les acides téréphtalique (T) et isophtalique (I). A titre d'exemples de diamines aliphatiques, on peut citer la tétraméthylène diamine, l'hexaméthylènediamine, la 1,10-décaméthylènediamine, la dodécaméthylènediamine, la triméthylhexaméthylène diamine. A titre d'exemple de diamines cycloaliphatiques, on peut citer les isomères des bis-(4-aminocyclohexyl)-méthane (BACM ou PACM), bis-(3-méthyl-4-aminocyclohexyl)méthane (BMACM ou MACM), et 2-2-bis-(3-méthyl-4-aminocyclohexyl)-propane (BMACP), l'isophoronediamine (IPDA), la 2,6-bis-(aminométhyl)-norbornane (BAMN) et la pipérazine (Pip).

Avantageusement, le copolymère selon l'invention comprend au moins un bloc PA à base de PA 4.4, PA 4.6, PA 4.9, PA 4.10, PA 4.12, PA 4.14, PA 4.16, PA 4.18, PA 4.36, PA 6.4, PA 6.6, PA 6.9, PA 6.10, PA 6.12, PA 6.13, PA 6.14, PA 6.16, PA 6.18, PA 6.36, PA 9.4, PA 9.6, PA 9.10, PA 9.12, PA 9.14, PA 9.18, PA 9.36, PA 10.4, PA 10.6, PA 10.9, PA 10.10, PA 10.12, PA 10.13, PA 10.14, PA 10.16, PA 10.18, PA 10.36, PA 10.T, PA BMACM.4, PA BMACM.6, PA BMACM.9, PA BMACM.10, PA BMACM.12, PA BMACM.14, PA BMACM.16, PA BMACM.18, PA BMACM.36, PA PACM.4, PA PACM.6, PA PACM.9, PA PACM.10, PA PACM. 12, PA PACM.14, PA PACM.16, PA PACM.18, PA PACM.36, PA Pip.4, PA Pip.6, PA Pip.9, PA Pip.10, PA Pip.12, PA Pip.14, PA Pip.16, PA Pip.18 et/ou PA Pip.36, et leurs mélanges.

Selon un deuxième type, les blocs polyamides résultent de la condensation d'un ou plusieurs acides alpha oméga aminocarboxyliques et/ou d'un ou plusieurs lactames ayant de 6 à 12 atomes de carbone en présence d'un diacide carboxylique ayant de 4 à 12 atomes de carbone ou d'une diamine. A titre d'exemples de lactames, on peut citer le caprolactame, l'oenantholactame et le lauryllactame. A titre d'exemples d'acide alpha omega amino carboxylique, on peut citer les acides aminocaproïque, amino-7-heptanoïque, amino-11- undécanoïque et amino-12-dodécanoïque.

Avantageusement, les blocs polyamides du deuxième type sont en polyamide 11, en polyamide 12 ou en polyamide 6.

Selon un troisième type, les blocs polyamides résultent de la condensation d'au moins un monomère du premier type avec au moins un monomère du deuxième type. En d'autres termes, les blocs polyamides résultent de la condensation d'au moins un acide alpha, oméga-aminocarboxylique (ou un lactame), avec au moins une diamine et un diacide carboxylique.

Dans ce cas, on prépare les blocs PA par polycondensation :
- de la ou des diamines aliphatiques, cycloaliphatiques ou aromatiques ayant X atomes de carbone ;
- du ou des diacides carboxyliques ayant Y atomes de carbone ; et
- du ou des comonomères {Z}, choisis parmi les lactames et les acides alpha-oméga aminocarboxyliques ayant Z atomes de carbone ;
- en présence d'un limiteur de chaîne choisi parmi les diacides carboxyliques ou diamines ou d'un excès de diacide ou de diamine utilisé comme unité structurale.

Avantageusement, on utilise comme limiteur de chaîne le diacide carboxylique ayant Y atomes de carbone, que l'on introduit en excès par rapport à la stoechiométrie de la ou des diamines.

Selon une autre variante, les blocs polyamides résultent de la condensation d'au moins deux acides alpha oméga aminocarboxyliques différents ou d'au moins deux lactames différents ayant de 6 à 12 atomes de carbone ou d'un lactame et d'un acide aminocarboxylique n'ayant pas le même nombre d'atomes de carbone en présence éventuelle d'un limiteur de chaîne.

A titre d'exemples de blocs polyamides du troisième type, on peut citer ceux formés par les polyamides (copolyamides) suivants :
P A 6/6.6 dans lequel 6 désigne du caprolactame et 6.6 désigne un monomère résultant de la condensation de l'hexaméthylènediamine avec de l'acide adipique.
P A 6.6/Pip.10112 dans laquelle 6.6 désigne un monomère résultant de la condensation de l'hexaméthylènediamine avec de l'acide adipique. Pip.10 désigne un monomère résultant de la condensation de la pipérazine avec l'acide sébacique. 12 désigne du lauryllactame.
P A 6.6/6.10/11/12 dans laquelle 6.6 désigne monomère résultant de la condensation de l'hexaméthylènediamine avec de l'acide adipique. 6.10 désigne un monomère résultant de la condensation de l'hexaméthylènediamine avec l'acide sébacique. 11 désigne l'acide amino-11-undécanoïque. 12 désigne du lauryllactame.

A titre d'exemples, on peut encore citer le PA 10.10/11, PA 6.10/11, PA10.12/11, PA 10.10/11/12, PA 6.10/10.10/11, PA 6.10/6.12/11, PA 6.10/6.12/10.10.

Les blocs polyéthers peuvent représenter 1 à 99%, et de préférence 5 à 90 % en poids du copolymère à blocs polyamides et polyéthers. La masse molaire Mn des blocs polyéther est comprise dans la gamme allant de 100 à 6 000 g/mol et de préférence de 200 à 3 000 g/mol, encore plus préférentiellement de 250 à 2000 g/mol.

La préparation des copolymères à bloc(s) polyamide et bloc(s) polyéther selon l'invention comprend tout moyen permettant d'accrocher les blocs polyamide (bloc PA) et les blocs polyéther (bloc PE) selon la présente invention. En pratique, on utilise essentiellement deux procédés l'un dit en 2 étapes, l'autre en une étape, ces deux procédés sont décrits dans la demande FR0856752. Avantageusement, les copolymères PEBA comprennent des blocs PA comprenant au moins l'un des polyamides suivants PA 11, PA 10.10, PA 10.12, PA 10.14, PA 10.18, PA 6.10, PA 6.12, PA 6.14, PA 6.18 comme composants majoritaires (pourcentage massique supérieur à 50% sur la masse totale de PA) et des blocs PE comprenant du PEG et/ou du PPG d'origine renouvelable comme composant majoritaire (pourcentage massique supérieur à 50% sur la masse totale de PE), et éventuellement du PO3G d'origine renouvelable comme autre composants des blocs PE du PEBA de l'invention. Des copolymères à blocs particulièrement préférés de l'invention sont le PA11-PEG, le PA10.10-PEG, le PA10.12-PEG, le PA10.14-PEG, le PA6.10- PEG, le PA6.12-PEG, le PA6.18-PEG, PA 11-PPG, PA10.10-PPG, PA10.12-PPG, PA10.14-PPG, PA6.10-PPG, PA6.12-PPG, PA6.18-PPG, PA11-PEG/PPG, PA10.10-PEG/PPG, PA10.12-PEG/PPG, PA10.14-PEG/PPG, PA6.10-PEG/PPG, PA6.12-PEG/PPG, et/ou PA6.18-PEG/PPG.

En particulier, le PA11- PEG, le PA11-PPG ou le PA11-PEG/PPG selon l'invention, d'origine totalement renouvelable, sont préférés.

Selon un deuxième mode de réalisation, le copolymère à blocs de la présente invention comprend au moins un bloc souple polyéther qui comprend au moins un PEG et/ou un PPG obtenu(s) au moins en partie à partir de matières premières d'origine renouvelable, et au moins un bloc polyester.

Par polyester au sens de l'invention on entend les produits de condensation des acides dicarboxyliques avec les diols, et en règle générale, tout polymère dont le squelette macromoléculaire contient des motifs de répétition contenant la fonction chimique ester.

Les blocs polyester (ci après abrégés blocs PES) sont habituellement fabriqués par polycondensation entre un acide dicarboxylique et un diol. Les acides carboxyliques appropriés comprennent ceux mentionnés ci-dessus utilisés pour former les blocs polyamide. Les diols appropriés comprennent les diols aliphatiques linéaires tels que l'éthylène glycol, le 1,3-propylène glycol, le 1,4-butylène glycol, le 1,6-hexylène glycol, les diols branchés tel que le néopentylglycol, le 3-méthylpentane glycol, le 1,2-propylène glycol, et les diols cycliques tel que le 1,4-bis(hydroxyméthyl)cyclohexane et le 1,4-cyclohexane-diméthanol.

Avantageusement, le copolymère à blocs de la présente invention forme un copolyétherester (abrégé COPE), c'est-à-dire un copolymère à blocs polyesters et blocs polyéthers.

Les COPE selon l'invention incluent donc tout TPE comprenant au moins un bloc polyéther (PE) issu au moins en partie d'oxyde d'éthylène et/ou d'oxyde de propylène comprenant du ¹⁴C , de préférence comprenant du polyéthylène glycol (ou PEG) et/ou du polypropylène glycol (ou PPG) issu au moins partiellement de matières renouvelables, et au moins un bloc polyester PES (homopolymère ou copolyester) issu de matières fossiles ou bien issu totalement ou partiellement (cas de polyesters mixtes) de matières premières renouvelables.

Les COPE comprennent des blocs polyéthers souples issus de polyétherdiols et des blocs polyesters rigides qui résultent de la réaction d'au moins un diacide carboxylique avec au moins un motif diol court allongeur de chaîne. Les blocs polyesters et les blocs polyéthers sont reliés par des liaisons esters résultant de la réaction des fonctions acides de l'acide dicarboxylique avec les fonctions OH du polyétherdiol. Le diol court allongeur de chaîne peut être choisi dans le groupe constitué du neopentylglycol, du cyclohexanediméthanol et des glycols aliphatiques de formule HO(CH₂)ₙOH dans laquelle n est un entier valant de 2 à 10. L'enchaînement des polyéthers et des diacides forme les blocs souples alors que l'enchaînement du glycol ou du butane diol avec les diacides forme les blocs rigides du copolyétherester.

Avantageusement, les diacides sont des acides dicarboxyliques aromatiques ayant de 8 à 14 atomes de carbone. Jusqu'à 50% en mole de l'acide aromatique dicarboxylique peut être remplacé par au moins un autre acide aromatique dicarboxylique ayant de 8 à 14 atomes de carbone, et/ou jusqu'à 20 % en mole peut être remplacé par un acide aliphatique dicarboxylique ayant de 2 à 14 atomes de carbone. A titre d'exemple d'acides aromatiques dicarboxyliques, on peut citer l'acide téréphtalique, isophtalique, bibenzoïque, naphtalène dicarboxylique, l'acide 4,4'-diphénylenedicarboxylique, l'acide bis(p-carboxyphényl) méthane, l'éthylène bis p-benzoïque acide, l'acide 1-4 tétraméthylène bis(p-oxybenzoïque), l'acide éthylène bis (p-oxybenzoïque), l'acide 1,3-triméthylène bis (p-oxybenzoique). A titre d'exemple de glycols, on peut citer l'éthylène glycol, le 1,3-triméthylène glycol, le 1,4-tétraméthylèneglycol, le 1,6-hexaméthylène glycol, le 1,3 propylène glycol, le 1,8 octaméthylèneglycol, le 1,10-décaméthylène glycol et le 1,4-cyclohexylène diméthanol.

Les copolymères à blocs polyesters et blocs polyéther sont des copolymères ayant des motifs polyéthers dérivés de polyétherdiols tels que définis précédemment, par exemple le polyéthylène glycol (PEG), le polypropylène glycol (PPG), le polytriméthylène glycol (PO3G) ou le polytétraméthylène glycol (PTMG), des motifs diacide carboxylique tels que l'acide téréphtalique et des motifs glycol (éthane diol) ou 1,4-butanediol. De tels copolyétheresters sont décrits dans les brevets EP402883 et EP405227. Ces polyétheresters peuvent également contenir des plastifiants et autres additifs bien connus de l'homme du métier.

Selon un troisième mode de réalisation, le copolymère à blocs de la présente invention comprend au moins un bloc souple polyéther issu au moins partiellement d'oxyde d'éthylène et/ou d'oxyde de propylène contenant du ¹⁴C , de préférence comprenant au moins un polyéthylène glycol et/ou un polypropylène glycol obtenu(s) au moins en partie à partir de matières premières d'origine renouvelable ; et au moins un bloc rigide polyuréthane.

Par polyuréthane (abrégé PU) au sens de l'invention on entend les produits issus de la réaction d'au moins un diisocyanate pouvant être choisi parmi les diisocyanates aromatiques (ex: MDI, TDI) et/ou les diisocyanates aliphatiques (ex: HDI ou hexaméthylènediisocyanate) avec au moins un diol court. Ce diol court allongeur de chaîne peut être choisi parmi les glycols cités plus haut dans la description des copolyétheresters. Les polyuréthanes entrant dans la composition des copolymères selon l'invention peuvent comprendre tous types de polyols, et en particulier ceux d'origine renouvelable, tels que les polyols issus de l'amidon (érythritol, sorbitol, maltitol, mannitol), les polyols dérivant de sucres tels que la saccharose (isomalt, xylitol), les polyols issus du maïs, du soja, du coton, du colza, du tournesol ou de l'arachide (glycerol, propylène glycol, éthylène glycol, coproduit de réaction de production de biodiésel). A titre d'autres exemples de polyols pouvant entrer dans la composition de ces polyuréthanes, on peut également citer le polyéthylène glycol (PEG), le poly(1,2-propylène glycol) (PPG), le poly(1,3-propylène glycol) (PO3G), le polytétraméthylène glycol (PTMG), qu'ils soient d'origine pétrochimique ou bien d'origine renouvelable. On utilisera avantageusement le PEG et/ou le PPG fabriqué(s) à partir de matières renouvelables.

Avantageusement, le copolymère à blocs de la présente invention forme un polyuréthane thermoplastique (abrégé TPU), c'est-à-dire un copolymère à blocs polyuréthanes et blocs polyéthers, aussi appelé polyétheruréthane.

Les TPU selon l'invention incluent donc tout TPE comprenant des blocs polyéther, ces derniers étant au moins en partie issus d'oxyde d'éthylène et/ou d'oxyde de propylène comprenant du ¹⁴C , en comprenant par exemple du PEG et/ou du PPG issu(s) au moins partiellement de matières renouvelables ; et des blocs PU (homopolymère ou copolyuréthane) issus de matières fossiles ou bien issus totalement ou partiellement (cas de polyuréthanes mixtes) de matières premières renouvelables, lesdits blocs PU pouvant également être issus d'oxyde d'éthylène et/ou de propylène comprenant du ¹⁴C , et comprendre par exemple du PEG et/ou du PPG issu(s) au moins partiellement de matières renouvelables.

Les polyétheruréthanes résultent de la condensation de blocs polyéthers souples qui sont des polyétherdiols et de blocs rigides polyuréthanes. Les blocs polyuréthanes et les blocs polyéthers sont reliés par des liaisons résultant de la réaction des fonctions isocyanates du polyuréthane avec les fonctions -OH du polyétherdiol.

Si les copolymères à blocs décrits ci-dessus comprennent généralement au moins un bloc polyéther souple et au moins un bloc rigide, il est évident que la présente invention couvre en fait tous les copolymères comprenant deux, trois, quatre (voire plus) blocs différents choisis parmi ceux décrits dans la présente description, dès l'instant qu'au moins un de ces blocs est issu d'au moins un monomère d'oxyde d'éthylène et/ou d'oxyde de propylène contenant du ¹⁴C.

Avantageusement, le copolymère selon l'invention est un copolymère segmenté à blocs comprenant trois types de blocs différents (nommé « tribloc » dans la présente description de l'invention), qui résultent de la condensation de plusieurs des blocs décrits ci-dessus. Ledit tribloc est de préférence choisi parmi les copolyétheresteramides, les copolyétheramideuréthanes, les copolyétheresteruréthanes, dans le(s)quel(s) :
- le pourcentage massique en bloc souple polyéther est supérieur à 20% ;
- le pourcentage massique en bloc rigide polyamide est supérieur à 10% ;
sur la masse totale de tribloc.

Le copolymère à blocs de l'invention peut aussi bien être utilisé seul qu'en mélange, ledit copolymère représentant en masse de 5 à 100%, de préférence de 5 à 70%, de préférence de 5 à 30%, sur la masse totale du mélange.

Le copolymère selon l'invention peut être additivé avec des stabilisants, des plastifiants, des lubrifiants, des charges naturelles ou organiques, des colorants, des pigments, de nacres, des agents antimicrobiens, des agents ignifugeants, des agents antistatiques, des agents modifiant la viscosité du copolymère, et/ou tout autre additif bien connu de l'homme du métier dans le domaine des polymères thermoplastiques.

La présente invention a également pour objet un procédé de préparation d'un copolymère tel que défini ci-dessus. Le procédé selon l'invention comprend l'étape de fourniture de polyéther (PE) issu au moins partiellement d'oxyde d'éthylène et/ou d'oxyde de propylène comprenant du ¹⁴C , et la transformation par synthèse en copolymère à blocs selon l'invention. Ladite transformation par synthèse conduit à un copolymère à blocs qui comprend au moins un bloc PEG et/ou PPG d'origine au moins partiellement renouvelable. De préférence, ledit PE comprend du PEG et/ou du PPG ayant une teneur en biocarbone d'au moins 1%.

En particulier, la préparation des copolymères à bloc(s) polyamide et bloc(s) polyéther selon l'invention comprend tout moyen permettant d'accrocher les blocs polyamide (bloc PA) et les blocs polyéther (bloc PE) selon la présente invention. En pratique, on utilise essentiellement deux procédés l'un dit en 2 étapes, l'autre en une étape.

Dans le procédé en une étape on mélange les précurseurs de polyamide, le limiteur de chaînes et le polyéther. Ainsi, dans le procédé en une étape on fabrique aussi des blocs polyamide. La polycondensation simultanée des blocs polyéthers et des précurseurs des blocs polyamides est conduite de préférence à une température de 180 à 300°C. On obtient alors un polymère ayant essentiellement des blocs polyéthers, des blocs polyamides de longueur très variable, mais aussi les différents réactifs ayant réagi de façon aléatoire qui sont répartis de façon statistique (aléatoire) le long de la chaîne polymère.

Que ce soit en une ou deux étapes il est avantageux d'opérer en présence d'un catalyseur. Par catalyseur, on entend tout produit permettant de faciliter la liaison des blocs polyamide et des blocs polyéther par estérification ou par amidification. Le catalyseur d'estérification est avantageusement un dérivé d'un métal choisi dans le groupe formé par le titane, le zirconium et l'hafnium ou encore un acide fort tel que l'acide phosphorique ou l'acide borique. On peut utiliser les catalyseurs décrits dans les brevets US 4 331 786, US 4 115 475, US 4 195 015, US 4 839 441, US 4 864 014, US 4 230 838 et US 4 332 920, WO 04 037898, EP 1262527, EP 1270211, EP 1136512, EP 1046675, EP 1057870, EP 1155065, EP 506495 et EP 504058.

Dans le procédé en deux étapes, on fabrique d'abord les blocs polyamides puis dans une deuxième étape on accroche les blocs polyamides et les blocs polyéthers. Les blocs polyétherdiols selon l'invention sont soit utilisés tels quels et copolycondensés avec des blocs polyamides à extrémités carboxyliques, soit ils sont aminés pour être transformés en polyéther diamines et condensés avec des blocs polyamides à extrémités carboxyliques. La méthode générale de préparation en deux étapes des copolymères PEBA ayant des liaisons ester entre les blocs PA et les blocs PE est connue et est décrite, par exemple, dans le brevet français FR 2 846 332. La méthode générale de préparation des copolymères PEBA de l'invention ayant des liaisons amide entre les blocs PA et les blocs PE est connue et décrite, par exemple dans le brevet européen EP 1 482 011.

La réaction de formation du bloc PA se fait habituellement entre 180 et 300°C, de préférence de 200 à 290°C, la pression dans le réacteur s'établit entre 5 et 30 bars, et on la maintient environ 2 à 3 heures. On réduit lentement la pression en mettant le réacteur à la pression atmosphérique, puis on distille l'eau excédentaire par exemple pendant une heure ou deux.

Le polyamide à extrémités acide carboxylique ayant été préparé, on ajoute ensuite le polyéther et un catalyseur. On peut ajouter le polyéther en une ou plusieurs fois, de même pour le catalyseur. Selon une forme avantageuse, on ajoute d'abord tout ou partie du polyéther, la réaction des extrémités OH du polyéther et des extrémités COOH du polyamide commence avec formation de liaisons ester et élimination d'eau. On élimine le plus possible l'eau du milieu réactionnel par distillation, puis on introduit le catalyseur pour achever la liaison des blocs polyamides et des blocs polyéthers. Cette deuxième étape s'effectue sous agitation, de préférence sous un vide d'au plus 100 mbar, de préférence d'au plus 50 mbar, de préférence d'au plus 20 mbar, de préférence au plus 10 mbar, à une température telle que les réactifs et les copolymères obtenus soient à l'état fondu. A titre d'exemple, cette température peut être comprise entre 100 et 300°C et le plus souvent entre 200 et 250°C. La réaction est suivie par la mesure du couple de torsion exercé par le polymère fondu sur l'agitateur ou par la mesure de la puissance électrique consommée par l'agitateur. La fin de la réaction est déterminée par la valeur du couple ou de la puissance cible.

On pourra également ajouter pendant la synthèse, au moment jugé le plus opportun, une ou plusieurs molécules utilisées comme anti-oxydant, par exemple l'Irganox® 1010 ou l'Irganox® 245.

Selon un mode de réalisation du procédé de l'invention, l'étape de fourniture de PE du procédé de l'invention comprend en outre une étape préalable de production de polyéther PEG et/ou PPG respectivement à partir d'oxyde d'éthylène (OE) et/ou d'oxyde de propylène (OP) ayant une teneur en biocarbone d'au moins 1%. Tout procédé connu de production de PEG et/ou de PPG respectivement à partir d'oxyde d'éthylène (OE) et/ou d'oxyde de propylène (OP) peut être employé pour la présente invention. Le volume A21, page 583, de l'Encyclopédie « Ullmann's Encyclopedia of Indusctrial Chemistry » décrit ces procédés.

A titre d'exemple, le polyéthylène glycol de faible poids moléculaire (poids moléculaire inférieur à 20000) est produit en faisant réagir de l'oxyde d'éthylène avec de l'eau, de l'éthylène glycol ou des oligomères d'éthylène glycol. La réaction est catalysée par des catalyseurs acides ou basiques, tels que des oxydes de métaux ou des oxydes alcalins. L'éthylène glycol et ses oligomères sont préférables à l'eau comme réactifs de départ, car ils permettent d'obtenir un polymère de distribution de poids moléculaire étroite (de faible polydispersité). La longueur de chaîne de polymère dépend du ratio entre les réactifs.

HOCH₂CH₂OH+n(CH₂CH₂O)→HO(CH₂CH₂O)ₙ₊₁H

Selon le type de catalyseur, le mécanisme de polymérisation peut être cationique ou anionique. Le mécanisme anionique est préféré car il permet d'obtenir un PEG de faible polydispersité. La polymérisation (polycondensation) de l'oxyde d'éthylène est un procédé exothermique.

L'oxyde de polyéthylène de haut poids moléculaire (supérieur à 20000) est synthétisé par polymérisation en suspension. Au cours du procédé de polycondensation, il est nécessaire de maintenir en solution la chaîne polymérique en croissance. Cette réaction est catalysée par des composés organo-métalliques du magnésium, de l'aluminium ou du calcium par exemple. Pour prévenir la coagulation des chaînes polymères hors de la solution, on utilise des additifs chélatant tels que la diméthylglyoxime.

Des catalyseurs alcalins tels que l'hydroxyde de sodium (NaOH), l'hydroxyde de potassium (KOH) ou le carbonate de sodium (Na₂CO₃) sont utilisés pour préparer du polyéthylène glycol de faible poids moléculaire.

Selon un autre mode de réalisation avantageux du procédé de l'invention, ladite étape de fourniture de PE, tel que le PEG et/ou le PPG, à partir respectivement d'oxyde d'éthylène (OE) et/ou d'oxyde de propylène (OP) du procédé de l'invention inclut en outre une étape préalable de production dudit oxyde d'éthylène à partir d'éthylène et/ou une étape préalable de production dudit oxyde de propylène à partir de propylène ; l'éthylène et/ou le propylène étant issu(s) de matières premières renouvelables.

La production d'oxyde d'éthylène à partir d'éthylène implique généralement l'oxydation directe de l'éthylène par l'air ou l'oxygène. On utilise de préférence un procédé d'oxydation directe de l'éthylène par l'oxygène, en présence d'un catalyseur entre 240 et 270 °C et sous une pression de 15 à 25 atm (1,5 à 2,5 Mpa) selon la réaction : C₂H₄+½O₂→C₂H₄O

Il existe deux procédés principaux d'obtention d'oxyde de propylène à partir de propylène : la voie classique ex-chlorhydrine et l'époxydation catalytique. Dans la voie classique ex-chlorhydrine, le propylène réagit avec du chlore en milieux aqueux pour donner une chlorhydrine qui est déhydrochlorée en présence de Ca(OH)₂. Dans l'époxydation catalytique, trois hydroperoxydes ont une importance industrielle : celui de tertiobutyle, celui d'éthylbenzène et le peroxyde de propylène. Par action sur le propylène, ils conduisent à l'oxyde de propylène.

Selon encore un autre mode de réalisation, ladite étape de fourniture de PE (PEG et/ou PPG) à partir d'éthylène et/ou de propylène du procédé de l'invention comprend en outre une étape préalable de production dudit éthylène et/ou dudit propylène à partir de matières premières renouvelables.

A titre d'exemple, la production d'éthylène à partir de matières premières renouvelables comprend les étapes suivantes :
a) fermentation de matières premières renouvelables et, éventuellement purification pour produire au moins un alcool choisi parmi l'éthanol et les mélanges d'alcools comprenant de l'éthanol ;
b) déshydratation de l'alcool obtenu pour produire, dans un premier réacteur, au moins un alcène choisi parmi l'éthylène et les mélanges d'alcènes comprenant de l'éthylène et, éventuellement purification de l'alcène pour obtenir de l'éthylène.

En tant que matières premières renouvelables pour la production d'éthylène ou de propylène, on pourra utiliser des matières végétales; des matières d'origine animale ou des matières d'origine végétale ou animale issues de matériaux récupérés (matériaux recyclés). Au sens de l'invention, les matières d'origine végétale contiennent au moins des sucres et/ou amidons. Les matières végétales contenant des sucres sont essentiellement la canne à sucre et la betterave sucrière, on peut également citer l'érable, le palmier-dattier, le palmier à sucre, le sorgho, l'agave américain ; les matières végétales contenant des amidons sont essentiellement les céréales et légumineuses comme le maïs, le blé, l'orge, le sorgho, le froment, le riz, la pomme de terre, le manioc, la patate douce, ou encore les algues. Parmi les matières issues de matériaux récupérés, on peut notamment citer les déchets végétaux ou organiques comprenant des sucres et/ou amidons. De préférence, les matières premières renouvelables sont des matières végétales.

En tant que matières premières renouvelables, on peut également utiliser de la cellulose ou de l'hémicellulose voire de la lignine qui, en présence des microorganismes adéquats, peuvent être transformées en matières comprenant du sucre. Parmi ces matières renouvelables, on compte la paille, le bois, le papier, qui peuvent provenir avantageusement de matériaux récupérés. Parmi les matières issues de matériaux récupérés, on peut notamment citer les déchets végétaux ou organiques comprenant des sucres et/ou des polysaccharides. Les listes présentées ci-dessus ne sont pas limitatives.

La fermentation des matières renouvelables s'effectue en présence d'un ou plusieurs microorganismes adéquats, ce microorganisme peut éventuellement avoir été modifié naturellement par une contrainte chimique ou physique, ou génétiquement on parle alors de mutant.

Classiquement le microorganisme utilisé pour la production d'éthylène est *Saccharomyces cerevisiae* ou un de ses mutants.

De préférence, l'étape de fermentation est suivie d'une étape de purification destinée à séparer l'éthanol des autres alcools.

A l'étape b) est mise en oeuvre la déshydratation du ou des alcools obtenus pour produire, dans un premier réacteur, au moins un alcène choisi parmi l'éthylène et les mélanges d'alcènes comprenant de l'éthylène, le produit secondaire de la déshydratation étant de l'eau.

Généralement, la déshydratation est effectuée à l'aide d'un catalyseur à base d'alpha-alumine comme le catalyseur commercialisé par EUROSUPPORT sous la dénomination commerciale ESM 110 ® (alumine trilobique non dopée contenant peu -environ 0,04%- de Na₂O résiduel).

Les conditions opératoires de la déshydratation font partie des connaissances générales de l'homme du métier, à titre indicatif, la déshydratation est généralement effectuée à une température de l'ordre de 400 °C.

Un autre avantage du procédé selon l'invention est son économie en énergie : les étapes de fermentation et déshydratation du procédé selon l'invention sont effectuées à des températures relativement basses, inférieures à 500°C, de préférence inférieures à 400°C. En comparaison, l'étape de craquage et de vapocraquage du pétrole en éthylène s'effectue à une température de l'ordre de 800°C. Cette économie d'énergie s'accompagne aussi d'une diminution du taux de CO₂ émis dans l'atmosphère.

De préférence une étape de purification est effectuée lors de l'étape a) ou lors de l'étape b). Les étapes éventuelles de purification (purification de(s) alcool(s) obtenus à l'étape a), purification de(s) alcène(s) obtenus à l'étape b)) sont avantageusement conduites par absorption sur des filtres classiques tels que tamis moléculaires, zéolithes, noir de carbone...). Si l'alcool obtenu à l'étape a) a été purifié de façon à isoler l'éthanol, l'alcène obtenu à l'étape b) est de l'éthylène. Si l'alcool obtenu à l'étape a) n'a pas été purifié, on obtient à l'issue de l'étape b) un mélange d'alcènes comprenant de l'éthylène.

Avantageusement, on effectue au moins une étape de purification lors de l'étape a) et/ou de l'étape b) afin d'obtenir de l'éthylène de pureté supérieure à 85% en poids, de préférence à 95% en poids, de manière préférée à 99% en poids et tout préférentiellement à 99,9% en poids. De manière particulièrement préférée, l'alcool obtenu à l'étape a) est purifié de façon à isoler l'éthanol, en conséquence l'alcène obtenu à l'étape b) est l'éthylène.

Les principales impuretés présentes dans l'éthylène issu de la déshydratation de l'éthanol sont l'éthanol, le propane et l'acétaldéhyde. Avantageusement, l'éthylène devra être purifié, c'est-à-dire que l'éthanol, le propane et l'acétaldéhyde devront être éliminés, pour pouvoir oxyder facilement l'éthylène. L'éthylène, l'éthanol, le propane et l'acétaldéhyde peuvent être séparés en mettant en oeuvre une ou plusieurs distillations à basse température. Les températures d'ébullition de ces composés sont les suivantes :

| composé | température d'ébullition (°C) |
|---|---|
| éthylène | -103,7 |
| propane | -42,1 |
| acétaldéhyde | 20,8 |
| éthanol | 75,5 |

L'éthylène, l'éthanol, le propane et l'acétaldéhyde sont refroidis à environ - 105°C, de préférence -103,7°C puis distillés pour extraire l'éthylène.

Un autre avantage du procédé selon la présente invention concerne les impuretés. Les impuretés présentes dans l'éthylène issu de la déshydratation de l'éthanol sont totalement différentes de celles présentes dans l'éthylène issu de craquage ou vapocraquage. En particulier parmi les impuretés présentes dans l'éthylène issu de craquage ou vapocraquage, on compte le dihydrogène et le méthane et ceci quelle que soit la composition de la charge initiale. Classiquement la séparation du dihydrogène et du méthane s'effectue après compression à 36 bar et refroidissement à environ -120°C. Dans ces conditions, le dihydrogène et le méthane, liquides, sont séparés dans le déméthaniseur ; puis l'éthylène est récupéré à 19 bar et -33°C. Le procédé selon la présente demande permet de s'affranchir de l'étape de séparation du dihydrogène et du méthane, et permet également de refroidir le mélange à -105°C à pression atmosphérique au lieu de -120°C à 36 bar. Le refroidissement de cette étape de séparation peut se faire aussi sous pression pour augmenter la température d'ébullition des composés à séparer (par exemple vers 20 bar et -35°C). Ces différences contribuent également à rendre le procédé selon l'invention plus économique (économie de matériel et économie d'énergie qui s'accompagne aussi d'une diminution du taux de CO₂ émis dans l'atmosphère).

Un autre avantage est que l'éthylène obtenu à l'étape b) du procédé selon l'invention ne comprend pas d'acétylène contrairement à l'éthylène obtenu par craquage ou vapocraquage. Or l'acétylène est très réactif et provoque des réactions secondaires d'oligomérisation, l'obtention d'éthylène sans acétylène est donc particulièrement avantageuse.

Un autre avantage est que le procédé selon l'invention peut être mis en oeuvre dans des unités de production localisées sur le lieu de production des matières premières. En outre, la taille des unités de production du procédé selon l'invention est beaucoup plus petite que la taille d'une raffinerie : les raffineries sont en effet de grosses installations situées généralement loin des centres de production des matières premières et alimentées par des pipelines.

A titre d'exemple, la production de propylène à partir de matières premières renouvelables comprend les étapes suivantes :
a') fermentation de matières premières renouvelables, et éventuellement purification, pour produire un alcool ou un mélange d'alcools, ledit alcool ou mélange d'alcools comprenant au moins de l'isopropanol et/ou au moins un mélange d'éthanol et de 1-butanol,
b') déshydratation de l'alcool ou du mélange d'alcools obtenus en vue de produire, dans au moins un premier réacteur, un alcène ou un mélange d'alcènes, ledit alcène ou mélange d'alcènes comprenant au moins du propylène et, éventuellement purification du mélange d'alcènes pour obtenir du propylène.

Ces étapes a) et b) sont notamment décrites dans la demande FR0858244.

En tant que matières premières renouvelables, on utilise celles déjà définies ci-dessus. Dans le cas des polysaccharides, la matière végétale mise en oeuvre se trouve généralement sous forme hydrolysée avant l'étape de fermentation. Cette étape préliminaire d'hydrolyse permet ainsi, par exemple, la saccharification de l'amidon pour le transformer en glucose, ou la transformation du sucrose en glucose.

La fermentation des matières renouvelables s'effectue en présence d'un ou plusieurs microorganismes adéquats, ce microorganisme peut éventuellement avoir été modifié naturellement par une contrainte chimique ou physique, ou génétiquement on parle alors de mutant.

Avantageusement le microorganisme utilisé pour la production de propylène est Clostridium beijerinckii ou un de ses mutants de préférence immobilisés sur un support du type fibre polymère ou calcium. Cette fermentation permet l'obtention d'un mélange d'alcools comprenant de l'éthanol, de l'isopropanol et du 1-butanol. Les alcools obtenus peuvent être extraits en continu à l'aide d'une membrane de pervaporation ; un avantage de l'utilisation de ce type de membrane est de permettre une meilleure conservation des microorganismes, car ceux-ci sont détruits lorsque leur concentration devient trop élevée.

D'autres micro-organismes pouvant être utilisés sont Clostridium aurantibutyricum ou Clostridium butylicum ainsi que leurs mutants. La fermentation de ces matières premières conduit essentiellement à la production d'isopropanol et/ou de butanols avec éventuellement de l'acétone.

Selon une première variante, l'alcool obtenu est essentiellement l'isopropanol.

L'étape de fermentation est avantageusement suivie d'une étape de purification par exemple une distillation destinée à séparer l'isopropanol des autres alcools.

Selon cette première variante, à l'étape b') est mise en oeuvre la déshydratation de l'isopropanol pour produire, dans un premier réacteur, au moins du propylène ou un mélange d'alcènes comprenant du propylène, le produit secondaire de la déshydratation étant de l'eau.

Généralement, la déshydratation est effectuée en présence d'oxygène et d'eau à l'aide d'un catalyseur à base d'alpha-alumine comme le catalyseur commercialisé par EUROSUPPORT sous la dénomination commerciale ESM110® (alumine trilobique non dopée contenant peu -environ 0,04%- de Na₂O résiduel).

Les conditions opératoires de la déshydratation font partie des connaissances générales de l'homme du métier, à titre indicatif, la déshydratation est généralement effectuée à une température de l'ordre de 400 °C.

Un avantage de ce procédé selon l'invention est son économie en énergie : les étapes de fermentation et déshydratation du procédé selon l'invention sont effectuées à des températures relativement basses, inférieures à 500°C, de préférence inférieures à 400°C, en comparaison l'étape de craquage et de vapocraquage du pétrole en propylène s'effectue à une température de l'ordre de 800°C.

Cette économie d'énergie s'accompagne aussi d'une diminution du taux de CO₂ émis dans l'atmosphère.

Selon une deuxième variante, qui peut être mise en oeuvre suite à une fermentation au moyen de Clostridium beijerinckii ou un de ses mutants, on obtient un mélange d'alcools comprenant au moins de l'éthanol et du 1-butanol.

Avantageusement, l'étape de fermentation est suivie d'une étape de purification par exemple une distillation destinée à séparer l'éthanol et le 1-butanol des autres alcools.

Selon cette deuxième variante, l'étape b') est mise en oeuvre au moyen d'une série de réacteurs :
- dans une première partie de la série de réacteurs (située en entrée de la série de réacteurs dans le sens du passage des fluides) est effectuée la déshydratation de l'éthanol et du 1-butanol en vue de produire au moins de l'éthylène et du 1-butène, cette déshydratation étant effectuée dans les mêmes conditions que la déshydratation de l'isopropanol décrite ci-dessus ;
- dans une seconde partie de cette première série de réacteurs (située dans la partie intermédiaire de la série de réacteurs) est effectuée une réaction d'hydro-isomérisation du 1-butène en 2-butène,
- dans une troisième partie de cette première série de réacteurs (située en sortie de la série de réacteurs dans le sens du passage des fluides) est effectuée la métathèse de l'éthylène et du 2-butène pour former du propylène.

Les détails des réactions d'hydro-isomérisation et de métathèse sont par exemple mentionnés dans la demande de brevet FR 2 880 018.

La réaction d'hydro-isomérisation du 1-butène en 2-butène s'effectue généralement à l'aide d'une composition catalytique comprenant un composé d'un métal de transition du groupe VIII et plus particulièrement du palladium ou du nickel. La composition catalytique peut également comprendre un sel d'ammonium et/ou de phosphonium quaternaire ce qui permet d'effectuer la réaction à une température relativement basse, en système fermé, semi-fermé ou en continu.

La réaction de métathèse est effectuée par passage des réactifs au contact d'un lit de catalyseur, la métathèse est en général réalisée en continu et comprend une phase de réaction et une phase de régénération. Les catalyseurs utilisés contiennent de l'oxyde de rhénium sur alumine ou un composé dérivé d'alumine comme par exemple une silice-alumine ou un oxyde de bore-alumine.

De manière préférée, le microorganisme utilisé est Clostridium beijerinckii ou un de ses mutants, ce microorganisme peut en effet être utilisé pour mettre en oeuvre la première variante et la deuxième variante, ainsi le procédé peut être mis en oeuvre en utilisant l'isopropanol et/ou la combinaison de l'éthanol et du 1-butanol.

Les étapes éventuelles de purification (purification de(s) alcool(s) obtenus à l'étape a'), purification de(s) alcène(s) obtenus à l'étape b')) sont avantageusement conduites par absorption sur des filtres classiques tels que tamis moléculaires, zéolithes, noir de carbone....

Avantageusement, on effectue au moins une étape de purification lors de l'étape a') et/ou de l'étape b') afin d'obtenir du propylène de degré de pureté suffisant pour effectuer une polymérisation ou une copolymérisation. On préférera obtenir du propylène de degré de pureté supérieure à 85% en poids, de préférence à 95% en poids, de manière préférée à 99% en poids et tout préférentiellement à 99,9% en poids. Les principales impuretés présentes dans le propylène issu de ces déshydratations sont l'acétone, le di-isopropyléther, l'acétaldéhyde, le 1-propanol et l'hydrogène.

Avantageusement, le propylène est purifié, c'est-à-dire que l'acétone, le diisopropyléther, l'acétaldéhyde, le 1-propanol et l'hydrogène devront être éliminés. L'hydrogène qui a une température d'ébullition très inférieure à celle du propylène peut être isolé en comprimant le gaz puis en le refroidissant légèrement, par exemple à 19 bars et -33°C.

Le propylène, l'acétone, le di-isopropyléther, l'acétaldéhyde, le 1-propanol peuvent être séparés en mettant en oeuvre une ou plusieurs distillations à basse température. Les températures d'ébullition à pression atmosphérique de ces composés sont les suivantes :

| composé | température d'ébullition (°C) |
|---|---|
| propylène | -47,7 |
| acétaldéhyde | 20,8 |
| acétone | 56 |
| diisopropyléther | 68 |
| 1-propanol | 97 |

Le propylène, l'acétone, le di-isopropyléther, l'acétaldéhyde, le 1-propanol sont refroidis à pression atmosphérique à environ -50°C, de préférence -47,7°C puis distillés pour extraire le propylène. On peut éventuellement faire cette distillation sous pression afin de pouvoir extraire le propylène à plus haute température.

Un autre avantage du procédé selon la présente invention concerne les impuretés. Les impuretés présentes dans le propylène issu de la déshydratation des alcools sont totalement différentes de celles présentes dans le propylène issu de craquage ou vapocraquage. En particulier parmi les impuretés présentes dans le propylène issu de craquage ou vapocraquage, on compte le méthylacéthylène et le propadiène.

Avec le procédé selon la présente invention, on obtient également du méthylacéthylène et propadiène mais ces composés sont alors présents dans des quantités nettement inférieures. Cette différence permet de limiter les risques liés au caractère très réactif du méthylacéthylène et également de limiter les réactions secondaires d'oligomérisation. Un autre avantage est que le procédé selon l'invention peut être mis en oeuvre dans des unités de production localisées sur le lieu de production des matières premières. En outre, la taille des unités de production du procédé selon l'invention est beaucoup plus petite que la taille d'une raffinerie : les raffineries sont en effet de grosses installations situées généralement loin des centres de production des matières premières et alimentées par des pipelines. Toutes ces différences contribuent à rendre le procédé selon l'invention plus économique (économie de matériel et économie d'énergie qui s'accompagne aussi d'une diminution du taux de CO2 émis dans l'atmosphère) que les procédés classiques d'obtention du propylène.

Les copolymères à blocs PEG obtenus selon le procédé de l'invention présentent de bonnes propriétés, en terme de densité, de reprise en eau et/ou de propriétés mécaniques, de propriétés antistatiques, de propriétés imper-respirantes et de propriétés d'étanchéité. Les copolymères à blocs PPG obtenus selon le procédé de l'invention présentent de bonnes propriétés, en terme de faible reprise en humidité, et en terme de stabilité des propriétés mécaniques vis-à-vis de l'humidité ambiante. Les copolymères comprenant à la fois des blocs PEG et PPG de l'invention présentent une combinaison de ces propriétés avantageuses. Le procédé de l'invention permet de moduler facilement ces propriétés en faisant varier le ratio PEG/PPG.

En définitive, le procédé selon l'invention permet d'obtenir un copolymère à blocs à partir d'oxyde d'éthylène et/ou d'oxyde de propylène d'origine renouvelable, et de performances identiques (voire meilleures) à celles des copolymères correspondants (même formule chimique) mais d'origine fossile. En définitive, le procédé de fabrication de copolymères à blocs de l'invention permet de diminuer, voire de supprimer complètement la consommation de matières premières d'origine pétrolière, et de faire appel à des matières premières issues de la culture de végétaux. Il permet en outre de diminuer les émissions de gaz à effets de serre.

L'invention concerne en particulier les objets suivants :
Objet 1- Copolymère à blocs issu d'au moins un monomère d'oxyde d'éthylène et/ou d'oxyde de propylène contenant du ¹⁴C déterminé selon la norme ASTM D6866.
Objet 2- Copolymère selon l'objet 1, comprenant au moins un bloc polyéther issu au moins partiellement d'oxyde d'éthylène et/ou d'oxyde de propylène contenant du ¹⁴C_{.}
Objet 3- Copolymère à blocs selon l'objet 1 ou 2 comprenant :
   - de 1 à 99% d'au moins un bloc polyéther souple issu au moins partiellement d'oxyde d'éthylène et/ou d'oxyde de propylène contenant du ¹⁴C , et
   - de 1 à 99% d'au moins un bloc rigide choisi parmi : les blocs polyamide, les blocs polyuréthane, les blocs polyester, et leurs mélanges.
Objet 4- Copolymère à blocs selon l'un quelconque des objets 1 à 3, dans lequel ledit au moins un bloc polyéther comprend au moins un polyéthylène glycol (PEG) et/ou polyropylène glycol (PPG) issu au moins partiellement de matières renouvelables.
Objet 5- Copolymère selon l'un quelconque des objets 3 ou 4, dans lequel :
   - la proportion massique dudit au moins un bloc souple représente de 5 à 95%, de préférence de 5 à 85%,
   - la proportion massique dudit au moins un bloc rigide représente de 5 à 95%, de préférence de 15 à 95%,
   sur la masse totale du copolymère.
Objet 6- Copolymère selon l'un quelconque des objets 3 à 5, dans lequel ledit au moins un bloc rigide est issu au moins partiellement de matières premières renouvelables.
Objet 7- Copolymère selon l'un quelconque des objets 3 à 6, dans lequel ledit au moins un bloc polyéther et/ou ledit au moins un bloc rigide est/sont issu(s) en totalité de matières renouvelables.
Objet 8- Copolymère selon l'un quelconque des objets précédents, comprenant une teneur en bio-carbone d'au moins 1%, qui correspond à un ratio isotopique de ¹⁴C/¹²C d'au moins 1,2. 10⁻¹⁴.
Objet 9- Copolymère selon l'un quelconque des objets précédents, comprenant une teneur en bio-carbone supérieure à 5%, de préférence supérieure à 10%, de préférence supérieure à 25%, de préférence supérieure à 50%, de préférence supérieure à 75%, de préférence supérieure à 90%, de préférence supérieure à 95%, de préférence supérieure à 98%, de préférence supérieure à 99%, avantageusement sensiblement égale à 100%.
Objet 10- Copolymère selon l'un quelconque des objets précédents, caractérisé en ce qu'il comprend au moins un bloc polyamide.
Objet 11- Copolymère selon l'objet 10, dans lequel le polyamide comprend un copolyamide.
Objet 12- Copolymère selon l'objet 10 ou 11, dans lequel ledit au moins un bloc polyamide comprend au moins une des molécules suivantes : acide amino-11-undécanoïque, acide n-heptylamino-11-undécanoïque, acide succinique, acide azélaïque, acide sébacique, acide dodécanedioïque, acide myristique, acide tétradécanedioïque, acide hexadécanedioïque, acide octadécanedioïque, butanediamine, pentanediamine, décaméthylènediamine, diacide(s) gras, dimère(s) d'acide gras et leurs mélanges.
Objet 13- Copolymère selon l'un quelconque des objets 10 à 12, dans lequel ledit au moins un bloc polyamide comprend au moins un monomère choisi parmi les monomères de polyamide suivants : 11, 5.4, 5.9, 5.10, 5.12, 5.13, 5.14, 5.16, 5.18, 5.36, 6.4, 6.9, 6.10, 6.12, 6.13, 6.14, 6.16, 6.18, 6.36, 10.4, 10.9, 10.10, 10.12, 10.13, 10.14, 10.16, 10.18, 10.36, 10.T, 12.4, 12.9, 12.10, 12.12, 12.13, 12.14, 12.16, 12.18, 12.36, 12.T et leurs mélanges.
Objet 14- Copolymère selon l'un quelconque des objets précédents, dans lequel ledit copolymère est un copolymère à blocs polyether et blocs polyamide (PEBA).
Objet 15- Copolymère selon l'un quelconque des objets précédents, dans lequel ledit copolymère comprend au moins un bloc polyester.
Objet 16- Copolymère selon l'objet 15, dans lequel le polyester comprend un copolyester.
Objet 17- Copolymère selon l'objet 15 ou 16, dans lequel ledit au moins un bloc polyester comprend au moins une des molécules suivantes : éthylène glycol, 1,3-propanediol, 1,4-butanediol, 1,10-décanediol, acide 2,5-furanedicarboxylique, acide succinique, acide azélaïque, acide sébacique, acide dodécanedioïque, acide myristique, acide tétradécanedioïque, acide hexadécanedioïque, acide octadécanedioique, diacide gras, acide gras dimérisés.
Objet 18- Copolymère selon l'un quelconque des objets 15 à 17, dans lequel ledit copolymère est un polyétherester.
Objet 19- Copolymère selon l'un quelconque des objets précédents, dans lequel ledit copolymère comprend au moins un bloc polyuréthane.
Objet 20- Copolymère selon l'objet 19, dans lequel le polyuréthane comprend un copolyuréthane.
Objet 21- Copolymère selon l'objet 19 ou 20, dans lequel ledit au moins un bloc polyuréthane est fabriqué à partir d'au moins un polyol d'origine renouvelable, choisi parmi les polyols suivants : polyols issus de l'amidon ; érythritol ; sorbitol ; maltitol ; mannitol ; polyols dérivant de sucres, du saccharose ; isomalt ; le xylitol ; polyols issus du maïs, du soja, du coton, du colza, du tournesol ou de l'arachide ; glycérol ; propylène glycol ; éthylène glycol ; les coproduits de réaction de production de biodiésel ; polyéthylène glycol (PEG), poly(1,2-propylène glycol) (PPG), poly(1,3-propylène glycol) (PO3G), polytétraméthylène glycol (PTMG).
Objet 22- Copolymère selon l'un quelconque des objets 19 à 21, dans lequel ledit copolymère est un polyétheruréthane.
Objet 23- Copolymère selon l'un quelconque des objets 4 à 22, caractérisé en ce que ledit au moins un bloc polyéther comprend en outre des polyéthers autres que PEG et/ou PPG, tels que le PO3G, le PTMG, le poly(3-méthyltétrahydrofurane) d'origine renouvelable ou d'origine fossile et/ou du PEG ou du PPG d'origine fossile.
Objet 24- Copolymère selon l'un quelconque des objets précédents, caractérisé en ce que ledit copolymère est un tribloc comprenant trois blocs différents, ledit tribloc étant choisi parmi les copolyétheresteramides, les copolyétheramideuréthanes, les copolyétheresteruréthanes, dans le(s)quel(s) :
   - le pourcentage massique en bloc souple polyéther est supérieur à 20% ;
   - le pourcentage massique en bloc rigide polyamide est supérieure à 10% ;
   sur la masse totale de tribloc.
Objet 25- Procédé de préparation de copolymère à blocs conforme à celui de l'un quelconque des objets précédents, comprenant l'étape de fourniture de polyéther issu au moins partiellement d'oxyde d'éthylène et/ou d'oxyde de propylène contenant du ¹⁴C et la transformation par synthèse en copolymère à blocs.
Objet 26- Procédé selon l'objet 25, dans lequel ledit polyéther comprend du polyéthylène glycol et/ou du polypropylène glycol ayant une teneur en biocarbone d'au moins 1%.
Objet 27- Procédé selon l'objet 25 ou 26, dans lequel l'étape de fourniture comprend une étape de production de polyéther à partir de d'oxyde d'éthylène et/ou d'oxyde de propylène ayant une teneur en biocarbone d'au moins 1%.
Objet 28- Procédé selon l'objet 27, dans lequel l'étape de fourniture comprend une étape de production dudit oxyde d'éthylène et/ou oxyde de propylène respectivement à partir d'éthylène et/ou propylène.
Objet 29- Procédé selon l'objet 28, dans lequel l'étape de fourniture comprend une étape de production dudit éthylène et/ou propylène à partir de biomasse végétale.
Objet 30- Procédé selon l'objet 29, dans lequel la production d'éthylène à partir de matières premières renouvelables comprend les étapes suivantes :
   a) fermentation de matières premières renouvelables et éventuellement purification pour produire au moins un alcool choisi parmi l'éthanol et les mélanges d'alcools comprenant de l'éthanol ;
   b) déshydratation de l'alcool obtenu pour produire, dans un premier réacteur, au moins un alcène choisi parmi l'éthylène et les mélanges d'alcènes comprenant de l'éthylène et, éventuellement purification de l'alcène pour obtenir de l'éthylène.
Objet 31- Procédé selon l'objet 28, dans lequel la production de propylène à partir de matières premières renouvelables comprend les étapes suivantes :
   a') fermentation de matières premières renouvelables, et éventuellement purification, pour produire un alcool ou un mélange d'alcools, ledit alcool ou mélange d'alcools comprenant au moins de l'isopropanol et/ou au moins un mélange d'éthanol et de 1-butanol,
   b') déshydratation de l'alcool ou du mélange d'alcools obtenus en vue de produire, dans au moins un premier réacteur, un alcène ou un mélange d'alcènes, ledit alcène ou mélange d'alcènes comprenant au moins du propylène et, éventuellement purification du mélange d'alcènes pour obtenir du propylène.
Objet 32- Utilisation de copolymère à blocs conforme à celui de l'un quelconque des objets 1 à 24, dans l'automobile, les textiles, les tissés, les non-tissés, l'habillement, les chaussures, les articles de sport, les articles de loisirs, l'électronique, le matériel informatique, les équipements de santé, les additifs industriels, l'emballage et/ou les produits ménagers.
Objet 33- Utilisation selon l'objet 32, dans laquelle le copolymère à blocs conforme à celui de l'un quelconque des objets 1 à 24 est utilisé dans les tableaux de bord, les airbags, les semelles de chaussure de sport, les balles de golf, les tubes à usage médical, les cathéters, les ballons d'angioplastie, les courroies péristaltiques, les bandes de courroie transporteuse, les produits imper-respirant, les additifs antistatiques, les peaux, et/ou les cuirs synthétiques, les films thermoplastiques, les films d'emballage.
Objet 34- Utilisation selon l'objet 32 ou 33 dans laquelle le copolymère à blocs conforme à celui de l'un quelconque des objets 1 à 24 est utilisé seul ou en mélange, ledit copolymère représentant en masse de 5 à 100%, de préférence de 5 à 70%, de préférence de 5 à 30%.
Objet 35- Copolymère selon l'objet 14, dans lequel ledit PEBA est à base de PA 11-PEG, PA10.10-PEG, PA10.12-PEG, PA10.14-PEG, PA6.10-PEG, PA6.12-PEG, PA6.18-PEG, PA 11-PPG, PA10.10-PPG, PA10.12-PPG, PA10.14-PPG, PA6.10-PPG, PA6.12-PPG, PA6.18-PPG, PA11-PEG/PPG, PA10.10-PEG/PPG, PA10.12-PEG/PPG, PA10.14-PEG/PPG, PA6.10-PEG/PPG, PA6.12-PEG/PPG, et/ou PA6.18-PEG/PPG, de préférence à base de PA11-PEG, PA11-PPG ou PA11-PEG/PPG.

## Revendications

1. Copolymère à blocs issu d'au moins un monomère d'oxyde d'éthylène et/ou d'oxyde de propylène contenant du ¹⁴C déterminé selon la norme ASTM D6866.

2. Copolymère selon la revendication 1, comprenant au moins un bloc polyéther issu au moins partiellement d'oxyde d'éthylène et/ou d'oxyde de propylène contenant du ¹⁴C ; dans lequel optionnellement ledit au moins un bloc polyéther comprend au moins un polyéthylène glycol (PEG) et/ou polypropylène glycol (PPG) issu au moins partiellement de matières renouvelables ; et dans lequel, optionnellement, ledit au moins un bloc polyéther comprend en outre des polyéthers autres que PEG et/ou PPG, tels que le PO3G, le PTMG, le poly(3-méthyltétrahydrofurane) d'origine renouvelable ou d'origine fossile et/ou du PEG ou du PPG d'origine fossile.

3. Copolymère à blocs selon la revendication 1 ou 2 comprenant :
- de 1 à 99% d'au moins un bloc polyéther souple issu au moins partiellement d'oxyde d'éthylène et/ou d'oxyde de propylène contenant du ¹⁴C , et
- de 1 à 99% d'au moins un bloc rigide choisi parmi : les blocs polyamide, les blocs polyuréthane, les blocs polyester, et leurs mélanges ;
et dans lequel optionnellement : .
- la proportion massique dudit au moins un bloc souple représente de 5 à 95%, de préférence de 5 à 85%,
- la proportion massique dudit au moins un bloc rigide représente de 5 à 95%, de préférence de 15 à 95%,
sur la masse totale du copolymère.

4. Copolymère selon la revendication 3, dans lequel ledit au moins un bloc rigide est issu au moins partiellement de matières premières renouvelables ; ou dans lequel ledit au moins un bloc polyéther et/ou ledit au moins un bloc rigide est/sont issu(s) en totalité de matières renouvelables.

5. Copolymère selon l'une quelconque des revendications précédentes, comprenant une teneur en bio-carbone d'au moins 1%, qui correspond à un ratio isotopique de ¹⁴C/¹²C d'au moins 1,2. 10⁻¹⁴, et comprenant optionnellement une teneur en bio-carbone supérieure à 5%, de préférence supérieure à 10%, de préférence supérieure à 25%, de préférence supérieure à 50%, de préférence supérieure à 75%, de préférence supérieure à 90%, de préférence supérieure à 95%, de préférence supérieure à 98%, de préférence supérieure à 99%, avantageusement sensiblement égale à 100%.

6. Copolymère selon l'une quelconque des revendications précédentes, **caractérisé en ce qu'**il comprend au moins un bloc polyamide ; dans lequel le polyamide comprend optionnellement un copolyamide ; dans lequel optionnellement ledit au moins un bloc polyamide comprend au moins une des molécules suivantes : acide amino-11-undécanoïque, acide n-heptylamino-11-undécanoïque, acide succinique, acide azélaïque, acide sébacique, acide dodécanedioïque, acide myristique, acide tétradécanedioïque, acide hexadécanedioïque, acide octadécanedioïque, butanediamine, pentanediamine, décaméthylènediamine, diacide(s) gras, dimère(s) d'acide gras et leurs mélanges ; et dans lequel également optionnellement ledit au moins un bloc polyamide comprend au moins un monomère choisi parmi les monomères de polyamide suivants : 11, 5.4, 5.9, 5.10, 5.12, 5.13, 5.14, 5.16, 5.18, 5.36, 6.4, 6.9, 6.10, 6.12, 6.13, 6.14, 6.16, 6.18, 6.36, 10.4, 10.9, 10.10, 10.12, 10.13, 10.14, 10.16, 10.18, 10.36, 10.T, 12.4, 12.9, 12.10, 12.12, 12.13, 12.14, 12.16, 12.18, 12.36, 12.T et leurs mélanges.

7. Copolymère selon l'une quelconque des revendications précédentes, dans lequel ledit copolymère est un copolymère à blocs polyether et blocs polyamide (PEBA) ; et dans lequel optionnellement ledit PEBA est à base de PA11-PEG, PA10.10-PEG, PA10.12-PEG, PA10.14-PEG, PA6.10-PEG, PA6.12-PEG, PA6.18-PEG, PA 11-PPG, PA10.10-PPG, PA10.12-PPG, PA10.14-PPG, PA6.10-PPG, PA6.12-PPG, PA6.18-PPG, PA11-PEG/PPG, PA10.10-PEG/PPG, PA10.12-PEG/PPG, PA10.14-PEG/PPG, PA6.10-PEG/PPG, PA6.12-PEG/PPG, et/ou PA6.18-PEG/PPG, de préférence à base de PA11-PEG, PA11-PPG ou PA11-PEG/PPG.

8. Copolymère selon l'une quelconque des revendications précédentes, dans lequel ledit copolymère comprend au moins un bloc polyester ; dans lequel optionnellement le polyester comprend un copolyester ; dans lequel optionnellement ledit au moins un bloc polyester comprend au moins une des molécules suivantes : éthylène glycol, 1,3-propanediol, 1,4-butanediol, 1,10-décanediol, acide 2,5-furanedicarboxylique, acide succinique, acide azélaïque, acide sébacique, acide dodécanedioïque, acide myristique, acide tétradécanedioïque, acide hexadécanedioïque, acide octadécanedioique, diacide gras, acide gras dimérisés ; et dans lequel, optionnellement également, ledit copolymère est un polyétherester.

9. Copolymère selon l'une quelconque des revendications précédentes, dans lequel ledit copolymère comprend au moins un bloc polyuréthane ; dans lequel optionnellement le polyuréthane comprend un copolyuréthane ; dans lequel optionnellement ledit au moins un bloc polyuréthane est fabriqué à partir d'au moins un polyol d'origine renouvelable, choisi parmi les polyols suivants : polyols issus de l'amidon ; érythritol ; sorbitol ; maltitol ; mannitol ; polyols dérivant de sucres, du saccharose ; isomalt ; le xylitol ; polyols issus du maïs, du soja, du coton, du colza, du tournesol ou de l'arachide ; glycérol ; propylène glycol ; éthylène glycol ; les coproduits de réaction de production de biodiésel ; polyéthylène glycol (PEG), poly(1,2-propylène glycol) (PPG), poly(1,3-propylène glycol) (PO3G), polytétraméthylène glycol (PTMG) ; et dans lequel optionnellement ledit copolymère est un polyétheruréthane.

10. Copolymère selon l'une quelconque des revendications précédentes, **caractérisé en ce que** ledit copolymère est un tribloc comprenant trois blocs différents, ledit tribloc étant choisi parmi les copolyétheresteramides, les copolyétheramideuréthanes, les copolyétheresteruréthanes, dans le(s)quel(s) :
- le pourcentage massique en bloc souple polyéther est supérieur à 20% ;
- le pourcentage massique en bloc rigide polyamide est supérieure à 10% ; sur la masse totale de tribloc.

11. Procédé de préparation de copolymère à blocs conforme à celui de l'une quelconque des revendications précédentes, comprenant l'étape de fourniture de polyéther issu au moins partiellement d'oxyde d'éthylène et/ou d'oxyde de propylène contenant du ¹⁴C et la transformation par synthèse en copolymère à blocs ; dans lequel de préférence ledit polyéther comprend du polyéthylène glycol et/ou du polypropylène glycol ayant une teneur en biocarbone d'au moins 1%.

12. Procédé selon la revendication 11, dans lequel l'étape de fourniture comprend une étape de production de polyéther à partir de d'oxyde d'éthylène et/ou d'oxyde de propylène ayant une teneur en biocarbone d'au moins 1% ; optionnellement l'étape de fourniture comprend une étape de production dudit oxyde d'éthylène et/ou oxyde de propylène respectivement à partir d'éthylène et/ou propylène ; et optionnellement encore l'étape de fourniture comprend une étape de production dudit éthylène et/ou propylène à partir de biomasse végétale.

13. Procédé selon la revendication 12, dans lequel la production d'éthylène à partir de matières premières renouvelables comprend les étapes suivantes :
a) fermentation de matières premières renouvelables et éventuellement purification pour produire au moins un alcool choisi parmi l'éthanol et les mélanges d'alcools comprenant de l'éthanol ;
b) déshydratation de l'alcool obtenu pour produire, dans un premier réacteur, au moins un alcène choisi parmi l'éthylène et les mélanges d'alcènes comprenant de l'éthylène et, éventuellement purification de l'alcène pour obtenir de l'éthylène ; et/ou
dans lequel la production de propylène à partir de matières premières renouvelables comprend les étapes suivantes :
a') fermentation de matières premières renouvelables, et éventuellement purification, pour produire un alcool ou un mélange d'alcools, ledit alcool ou mélange d'alcools comprenant au moins de l'isopropanol et/ou au moins un mélange d'éthanol et de 1-butanol,
b') déshydratation de l'alcool ou du mélange d'alcools obtenus en vue de produire, dans au moins un premier réacteur, un alcène ou un mélange d'alcènes, ledit alcène ou mélange d'alcènes comprenant au moins du propylène et, éventuellement purification du mélange d'alcènes pour obtenir du propylène.

14. Utilisation de copolymère à blocs conforme à celui de l'une quelconque des revendications 1 à 10, dans l'automobile, les textiles, les tissés, les non-tissés, l'habillement, les chaussures, les articles de sport, les articles de loisirs, l'électronique, le matériel informatique, les équipements de santé, les additifs industriels, l'emballage et/ou les produits ménagers ; et de préférence dans les tableaux de bord, les airbags, les semelles de chaussure de sport, les balles de golf, les tubes à usage médical, les cathéters, les ballons d'angioplastie, les courroies péristaltiques, les bandes de courroie transporteuse, les produits imper-respirant, les additifs antistatiques, les peaux, et/ou les cuirs synthétiques, les films thermoplastiques, les films d'emballage.

15. Utilisation selon la revendication 14 dans laquelle le copolymère à blocs conforme à celui de l'une quelconque des revendications 1 à 10 est utilisé seul ou en mélange, ledit copolymère représentant en masse de 5 à 100%, de préférence de 5 à 70%, de préférence de 5 à 30%.
